# EUROPÄISCHE PATENTSCHRIFT

(11) **EP 2 528 523 B1**
(45) Veröffentlichungstag und Bekanntmachung des Hinweises auf die Patenterteilung: **08.11.2017**
(21) Anmeldenummer: 10798801.6
(22) Anmeldetag: 21.12.2010
(51) Int. Cl.: A61B 17/70, A61B 17/00, A61B 90/00

(54) **CHIRURGISCHES INSTRUMENTARIUM**
SURGICAL INSTRUMENT
INSTRUMENT CHIRURGICAL

(30) Priorität: 27.01.2010 DE 102010000230
(43) Veröffentlichungstag der Anmeldung: 05.12.2012
(73) Patentinhaber: Aesculap AG, 78532 Tuttlingen (DE)
(72) Erfinder: HAAS, Alexander, 78166 Donaueschingen (DE)
(74) Vertreter: Hoeger, Stellrecht & Partner Patentanwälte mbB
(86) Internationale Anmeldenummer: PCT/EP2010/070361
(87) Internationale Veröffentlichungsnummer: WO 2011/091918

(56) Entgegenhaltungen:
- DE-U1-202008 009 344
- GB-A- 2 436 292
- US-A1- 2005 055 031
- US-A1- 2008 108 990
- US-A1- 2009 138 055

## Beschreibung

Die Erfindung betrifft ein chirurgisches Instrumentarium, umfassend ein Implantat zur gegenseitigen Abstützung eines oberen Dornfortsatzes eines ersten Wirbelkörpers mittels einer oberen Stützfläche und eines unteren Dornfortsatzes eines zweiten Wirbelkörpers mittels einer unteren Stützfläche, wobei das Implantat eine erste Implantatkomponente und eine relativ zu dieser entlang einer Spannrichtung beweglich ausgebildete zweite Implantatkomponente umfasst, um das Implantat ausgehend von einer Grundstellung in einer quer zur Spannrichtung ausgerichteten Spreizrichtung in eine Spreizstellung zu überführen, in der die obere Stützfläche und die untere Stützfläche einen größeren Abstand voneinander aufweisen als in der Grundstellung, und wobei das Implantat mindestens ein einbringseitiges oberes Stützelement und mindestens ein einbringseitiges unteres Stützelement zum einbringseitigen lateralen Abstützen des oberen Dornfortsatzes bzw. des unteren Dornfortsatzes in der Spreizstellung des Implantats umfasst.

Außerdem wird ein chirurgisches Instrumentarium mit einer Handhabungseinrichtung zum Halten eines Implantates beschrieben. Ein Implantat der eingangs genannten Art ist in der DE 20 2008 009 344 U1 beschrieben. Es kann in der Grundstellung durch einen unilateralen Zugang von einer Einbringseite in den Körper eingebracht werden und im Zwischenwirbelraum zwischen den Dornfortsätzen positioniert werden. Durch Überführen des Implantates von der Grundstellung in die Spreizstellung vergrößern die Stützflächen ihren Abstand voneinander, so dass die auf den Stützflächen ruhenden Dornfortsätze mehr oder weniger auseinander geschoben werden können, zur Stabilisierung benachbarter Wirbelkörper relativ zueinander. Zur lateralen Stabilisierung des oberen und des unteren Wirbelkörpers umfasst das Implantat mindestens ein oberes Stützelement sowie ein unteres Stützelement, die jeweils einbringseitig, d.h. von der Seite, von der das Implantat in den Körper eingebracht wird, die Dornfortsätze abstützen können. Die Stützelemente werden ebenfalls beim Überführen des Implantates von der Grundstellung in die Spreizstellung relativ zueinander gespreizt, wobei sie sich jeweils ausgehend vom Zwischenwirbelraum den Dornfortsätzen annähern. In entsprechender Weise kann das Implantat mindestens ein oberes Stützelement sowie mindestens ein unteres Stützelement umfassen, die körperseitig lateral den oberen bzw. den unteren Dornfortsatz abstützen können, d.h. von der der Einbringseite abgewandten Seite der Dornfortsätze.

Aufgabe der vorliegenden Erfindung ist es, ein gattungsgemäßes chirurgisches Instrumentarium so weiterzubilden, dass es eine verbesserte Handhabbarkeit aufweist.

Diese Aufgabe wird bei einem chirurgischen Instrumentarium der eingangs genannten Art erfindungsgemäß dadurch gelöst, dass das Instrumentarium eine Fixiereinrichtung für das Implantat umfasst zum Spreizen des mindestens einen einbringseitigen oberen Stützelementes und des mindestens einen einbringseitigen unteren Stützelementes relativ zueinander entlang der Spreizrichtung, mittels welcher Fixiereinrichtung das Implantat ausgehend von der Grundstellung in eine Einbringstellung bringbar ist, in der das mindestens eine einbringseitige obere Stützelement und das mindestens eine einbringseitige untere Stützelement einen größeren Abstand relativ zueinander aufweisen als in der Grundstellung, und aus der das Implantat durch Bewegen der zweiten Implantatkomponente relativ zur ersten Implantatkomponente entlang der Spannrichtung in die Spreizstellung überführbar ist. Vorteilhafte Weiterbildungen der Erfindung sind Gegenstand der abhängigen Ansprüche. Mittels der Fixiereinrichtung kann das Implantat ausgehend von einer Grundstellung in eine Einbringstellung gebracht werden. Das mindestens eine einbringseitige obere Stützelement und das mindestens eine einbringseitige untere Stützelement geraten dadurch relativ zueinander in einen Abstand, der größer ist als ihr Abstand voneinander in der Grundstellung des Implantates. Bei geeigneter Dimensionierung der Fixiereinrichtung kann ein Abstand zwischen einem oberen Stützelement und einem unteren Stützelement erzielt werden, der größer ist als der Abstand des oberen und des unteren Dornfortsatzes voneinander. Dies führt dazu, dass bereits beim Einbringen des Implantates in der Einbringstellung in den Zwischenwirbelraum das mindestens eine einbringseitige obere Stützelement zur Anlage an den oberen Dornfortsatz geraten kann und das mindestens eine einbringseitige untere Stützelement zur Anlage an den unteren Dornfortsatz, ohne dass hierfür ein Überführen des Implantates in die Spreizstellung erforderlich ist. Die Stützelemente bilden somit Anschläge für die Dornfortsätze aus, so dass die maximale Einbringtiefe des Implantates in den Zwischenwirbelraum dadurch begrenzt ist. Dies erleichtert einem Operateur die Handhabung des Implantates, und die Stützelemente können ungefähr an derjenigen Stelle zur Anlage an die Dornfortsätze geraten, die sie in der Spreizstellung des Implantates ebenfalls einnehmen können. Dies bedeutet, dass sich das Implantat relativ zu den Dornfortsätzen verbessert positionieren lässt. Darüber hinaus wird sichergestellt, dass ein Operateur das Implantat nicht zu tief in den Zwischenwirbelraum einbringt, es kann daher weniger invasiv gearbeitet werden, und eine Verletzungsgefahr für den Patienten ist verringerbar.

Die Fixiereinrichtung umfasst vorzugsweise eine Aufnahme, in der das Implantat, zumindest teilweise, gehalten ist. Ergänzend oder alternativ kann das Implantat eine Aufnahme umfassen, in der die Fixiereinrichtung, zumindest teilweise, gehalten ist. Die Fixiereinrichtung kann dadurch mit dem Implantat verbunden werden, damit dieses von der Grundstellung in die Einbringstellung gebracht werden kann.

Günstig ist es, wenn die Fixiereinrichtung mindestens einen entgegen der Spreizrichtung wirksamen ersten oberen Anschlag für das mindestens eine einbringseitige obere Stützelement oder einen dieses umfassenden Träger umfasst oder ausbildet und/oder wenn die Fixiereinrichtung mindestens einen entgegen der Spreizrichtung wirksamen ersten unteren Anschlag für das mindestens eine einbringseitige untere Stützelement oder einen dieses umfassenden Träger umfasst oder ausbildet. Dies gibt eine einfache konstruktive Möglichkeit, das obere Stützelement und das untere Stützelement gegeneinander zu spreizen, um ihren Abstand voneinander in der Einbringstellung des Implantates im Vergleich zur Grundstellung des Implantates zu vergrößern.

Von Vorteil ist es, wenn das mindestens eine einbringseitige obere Stützelement oder der es umfassende Träger in der Einbringstellung des Implantates an dem mindestens einen ersten oberen Anschlag anliegt und/oder wenn das mindestens eine einbringseitige untere Stützelement oder der es umfassende Träger in der Einbringstellung des Implantates an dem mindestens einen ersten unteren Anschlag anliegt. Dadurch lässt sich eine zuverlässige Spreizung des oberen Stützelementes und des unteren Stützelementes relativ zueinander sicherstellen. Der erste obere und/oder der erste untere Anschlag umfassen hierfür jeweils eine Anlagefläche für das jeweilige Spreizelement oder den jeweiligen Träger.

Günstig ist es, wenn die Fixiereinrichtung mindestens einen in der Spreizrichtung wirksamen zweiten oberen Anschlag für das mindestens eine einbringseitige obere Stützelement oder einen dieses umfassenden Träger umfasst oder ausbildet und/oder wenn die Fixiereinrichtung mindestens einen in der Spreizrichtung wirksamen zweiten unteren Anschlag für das mindestens eine einbringseitige untere Stützelement oder einen dieses umfassenden Träger umfasst oder ausbildet. Dadurch lässt sich auf einfache konstruktive Weise sicherstellen, dass in der Einbringstellung das obere und das untere Stützelement längs der Spreizrichtung einen maximalen Abstand, der durch den Abstand des zweiten oberen Anschlages und des zweiten unteren Anschlages voneinander gegeben ist, nicht überschreiten. Dies ermöglicht es, eine Beschädigung des Implantates in der Einbringstellung zu vermeiden.

Von Vorteil ist es, wenn das mindestens eine einbringseitige obere Stützelement oder der es umfassende Träger in der Einbringstellung des Implantates an dem mindestens einen zweiten oberen Anschlag anliegt und/oder wenn das mindestens eine einbringseitige untere Stützelement oder der es umfassende Träger in der Einbringstellung des Implantates an dem mindestens einen zweiten unteren Anschlag anliegt. Dadurch kann auf zuverlässige Weise sichergestellt werden, dass das obere Stützelement und das untere Stützelement entlang der Spreizrichtung nicht so weit gegeneinander gespreizt werden, dass das Implantat beschädigt wird. Der zweite obere und/oder der zweite untere Anschlag umfassen hierfür jeweils eine Anlagefläche für das jeweilige Stützelement oder den jeweiligen Träger.

Insbesondere kann vorgesehen sein, dass in der Einbringstellung des Implantates das mindestens eine einbringseitige obere Stützelement formschlüssig zwischen dem mindestens einen ersten oberen Anschlag und dem mindestens einen zweiten oberen Anschlag angeordnet ist und dass das mindestens eine einbringseitige untere Stützelement formschlüssig zwischen dem mindestens einen ersten unteren Anschlag und dem mindestens einen zweiten unteren Anschlag angeordnet ist, um eine zuverlässige Fixierung des Implantates sicherzustellen.

Günstig ist es, wenn die Fixiereinrichtung mindestens ein einbringseitiges oberes Knochenanschlagelement für den oberen Dornfortsatz umfasst oder ausbildet und/oder wenn die Fixiereinrichtung mindestens ein einbringseitiges unteres Knochenanschlagelement für den unteren Dornfortsatz umfasst oder ausbildet. Dies erleichtert die Handhabung des Implantates. Beim Einbringen des Implantates in den Zwischenwirbelraum kann sich dieses nicht nur mittels des oberen und des unteren Stützelementes an den oberen bzw. unteren Dornfortsatz anlegen, sondern auch mittels der Fixiereinrichtung. Dadurch ist eine zuverlässigere Positionierung des Implantates relativ zu den Dornfortsätzen erzielbar.

In der Einbringstellung des Implantates sind das obere Knochenanschlagelement und das untere Knochenanschlagelement relativ zueinander günstigerweise weiter beabstandet als das einbringseitige obere Stützelement und das einbringseitige untere Stützelement, um das Hantieren mit dem Implantat im Zwischenwirbelraum zu erleichtern.

Es kann vorgesehen sein, dass die erste Implantatkomponente das mindestens eine einbringseitige obere Stützelement und das mindestens eine einbringseitige untere Stützelement umfasst. Dies hat sich in der Praxis als vorteilhaft für eine einfachere Konstruktion des Implantates erwiesen.

Alternativ oder ergänzend kann vorgesehen sein, dass die zweite Implantatkomponente mindestens ein einbringseitiges oberes Stützelement und/oder mindestens ein einbringseitiges unteres Stützelement umfasst.

Vorzugsweise umfasst das Implantat mindestens ein körperseitiges oberes Stützelement und mindestens ein körperseitiges unteres Stützelement zum körperseitigen lateralen Abstützen des oberen Dornfortsatzes bzw. des unteren Dornfortsatzes in der Spreizstellung des Implantates. Wie bereits erwähnt, wird "körperseitig" vorliegend als diejenige laterale Seite der Dornfortsätze angesehen, die derjenigen lateralen Seite der Dornfortsätze abgewandt ist, auf die gerichtet das Implantat in den Körper eingebracht wird. Das obere Stützelement und das untere Stützelement ermöglichen körperseitig ein seitliches Abstützen des oberen bzw. des unteren Dornfortsatzes und dadurch auch eine zuverlässigere Abstützung derselben relativ zueinander.

Es kann vorgesehen sein, dass die zweite Implantatkomponente das mindestens eine körperseitige obere Stützelement und das mindestens eine körperseitige untere Stützelement umfasst. Dies hat sich in der Praxis als vorteilhaft für eine einfachere konstruktive Ausgestaltung des Implantates herausgestellt.

Alternativ oder ergänzend kann vorgesehen sein, dass die erste Implantatkomponente mindestens ein körperseitiges oberes Stützelement und/oder mindestens ein körperseitiges unteres Stützelement umfasst.

Bevorzugt umfasst das chirurgische Instrumentarium eine Kopplungseinrichtung mit mindestens einem mit einem einbringseitigen Stützelement gekoppelten ersten Kopplungselement sowie mit mindestens einem mit einem körperseitigen Stützelement gekoppelten zweiten Kopplungselement, das in der Einbringstellung des Implantates mit dem mindestens einen ersten Kopplungselement zusammenwirkt zur Verringerung des Abstandes des mindestens einen körperseitigen oberen Stützelementes und des mindestens einen körperseitigen unteren Stützelementes voneinander relativ zu deren Abstand voneinander in der Grundstellung des Implantates. In der Einbringstellung des Implantates weisen das mindestens eine einbringseitige obere Stützelement und das mindestens eine einbringseitige untere Stützelement voneinander einen größeren Abstand auf als in der Grundstellung des Implantates. Dies kann mittels der Fixiereinrichtung sichergestellt werden. Bei der vorliegenden Ausführungsform ist es mittels der zusätzlichen Kopplungseinrichtung weiter möglich, den Abstand des mindestens einen körperseitigen oberen Stützelementes und des mindestens einen körperseitigen unteren Stützelementes voneinander in der Einbringstellung des Implantates relativ zu deren Abstand voneinander in der Grundstellung des Implantates zu verringern. Dies gibt die Möglichkeit, die Einbringhöhe des Implantates mittels der Fixiereinrichtung und der Kopplungseinrichtung zu verringern. Dadurch wird einem Operateur das Einbringen des Implantates in den Zwischenwirbelraum erleichtert. Außerdem ist die Gefahr verringerbar, dass der Operateur beim Einbringen des Implantates unerwünschterweise an den Dornfortsätzen anstößt oder an anderen Körperstrukturen, wie z.B. Bändern, Sehnen oder Muskeln. Dies erleichtert die Handhabung des Implantates erheblich. Um ein einbringseitiges Stützelement mit einem körperseitigen Stützelement zu koppeln, umfasst die Kopplungseinrichtung das mindestens eine erste Kopplungselement und das mindestens eine zweite Kopplungselement, die in der Einbringstellung des Implantates zusammenwirken.

Das erste und/oder das zweite Kopplungselement kann mit dem einbringseitigen bzw. dem körperseitigen Stützelement beispielsweise verbunden sein, insbesondere einstückig, um mit ihm gekoppelt zu sein.

Günstig ist es, wenn das Implantat die Kopplungseinrichtung umfasst oder ausbildet, denn dies erlaubt es, dem chirurgischen Instrumentarium eine einfachere Konstruktion zu verleihen.

Es kann insbesondere vorgesehen sein, dass die erste Implantatkomponente das mindestens eine erste Kopplungselement umfasst und dass die mindestens eine zweite Implantatkomponente das mindestens eine zweite Kopplungselement umfasst.

Vorzugsweise umfasst die Kopplungseinrichtung ein mit einem einbringseitigen oberen Stützelement gekoppeltes erstes Kopplungselement und ein mit diesem zusammenwirkendes und mit einem körperseitigen unteren Stützelement gekoppeltes zweites Kopplungselement. Entlang der Spreizrichtung ist in der Einbringstellung des Implantates die Wirkung der Fixiereinrichtung zusammen mit der Kopplungseinrichtung in Bezug auf das einbringseitige obere Stützelement und das körperseitige untere Stützelement gegenläufig. Je weiter das einbringseitige obere Stützelement unter der Wirkung der Fixiereinrichtung relativ zu einem einbringseitigen unteren Stützelement gespreizt wird, desto näher gelangt das körperseitige untere Stützelement an ein körperseitiges oberes Stützelement. Es hat sich in der Praxis gezeigt, dass die Kopplungseinrichtung bei dieser Ausführungsform eine einfache Konstruktion aufweisen kann und eine zuverlässige Funktion der Kopplungseinrichtung sichergestellt werden kann.

In entsprechender Weise ist es zur Erzielung desselben Vorteils günstig, wenn die Kopplungseinrichtung ein mit einem einbringseitigen unteren Stützelement gekoppeltes erstes Kopplungselement und ein mit diesem zusammenwirkendes und mit einem körperseitigen oberen Stützelement gekoppeltes zweites Kopplungselement umfasst.

Vorzugsweise bilden das mindestens eine erste Kopplungselement und/oder das mindestens eine zweite Kopplungselement zumindest teilweise eine Stützfläche für einen Dornfortsatz aus. Das erste und/oder das zweite Kopplungselement können dadurch eine zusätzliche Funktion ausüben. Dies erlaubt es, dem Implantat eine kompaktere Bauform zu verleihen.

Günstig ist es, wenn die Kopplungseinrichtung ein erstes Kopplungselement für ein einbringseitiges oberes Stützelement und ein erstes Kopplungselement für ein einbringseitiges unteres Stützelement umfasst, die mittels der Fixiereinrichtung in der Einbringstellung des Implantates längs der Spreizrichtung weiter relativ zueinander gespreizt sind als in der Grundstellung des Implantates. Dadurch ist die Möglichkeit gegeben, zwei zweite Kopplungselemente, die mit den ersten Kopplungselementen zusammenwirken, längs der Spreizrichtung einander anzunähern, beispielsweise dann, wenn sich jeweils ein erstes Kopplungselement und ein zweites Kopplungselement längs der Spreizrichtung gegenseitig aufeinander abstützen. Wenn von den zweiten Kopplungselementen eines mit einem körperseitigen oberen Stützelement gekoppelt ist und eines mit einem körperseitigen unteren Stützelement, ermöglicht dies, das körperseitige obere und das körperseitige untere Stützelement längs der Spreizrichtung einander anzunähern.

Die Spreizung der ersten Kopplungselemente relativ zueinander wird mittels der Fixiereinrichtung erzielt, beispielsweise unter der Wirkung des vorstehend erwähnten ersten oberen Anschlags für ein einbringseitiges oberes Stützelement sowie des dem ersten unteren Anschlags für ein einbringseitiges unteres Stützelement.

Insbesondere sind die zwei ersten Kopplungselemente relativ zueinander längs der Spreizrichtung vorgespannt.

Vorzugsweise sind das mindestens eine erste Kopplungselement und/oder das mindestens eine zweite Kopplungselement elastisch verformbar ausgebildet, denn dadurch kann eine zuverlässigere Funktion der Kopplungseinrichtung und des Implantates sichergestellt werden.

Bei einer vorteilhaften Ausgestaltung des Instrumentariums hat es sich als günstig erwiesen, wenn das mindestens eine erste Kopplungselement als ein das einbringseitige Stützelement an einem freien Ende umfassender erster Träger ausgebildet ist, der ein längs der Spreizrichtung wirksames Aufgleitelement für das mindestens eine zweite Kopplungselement ausbildet, und/oder wenn das mindestens eine zweite Kopplungselement als ein das körperseitige Stützelement an einem freien Ende umfassender zweiter Träger ausgebildet ist, der ein längs der Spreizrichtung wirksames Aufgleitelement für das mindestens eine erste Kopplungselement ausbildet. Beim Überführen des Implantates von der Einbringstellung in die Spreizstellung können ein erstes Kopplungselement und ein zweites Kopplungselement jeweils aneinander aufgleiten und sich gegenseitig abstützen, wobei sie für das jeweils andere Kopplungselement ein Aufgleitelement ausbilden. Dieses Aufgleiten ist längs der Spreizrichtung wirksam. Das einbringseitige Stützelement und das körperseitige Stützelement, sei es für den oberen Dornfortsatz oder für den unteren Dornfortsatz, können dadurch jeweils entlang der Spreizrichtung bewegt werden, bis sie ihre Position in der Spreizstellung des Implantates einnehmen können. Dies ermöglicht eine zuverlässige Funktion des Implantates bei einer zugleich einfachen Konstruktion desselben.

Von Vorteil ist es, wenn das chirurgische Instrumentarium mindestens ein Blockierelement umfasst zum Verhindern einer Bewegung eines körperseitigen Stützelementes entlang der Spreizrichtung in der Einbringstellung des Implantates. Dadurch kann verhindert werden, dass das mindestens eine körperseitige obere Stützelement und das mindestens eine körperseitige untere Stützelement nicht unerwünschterweise in der Einbringstellung des Implantates ihren Abstand voneinander vergrößern. Dadurch kann die Einbringhöhe des Implantates vor dessen Überführen in die Spreizstellung konstant gehalten werden. Eine Gefahr, dass ein körperseitiges Stützelement unerwünschterweise einen Dornfortsatz oder eine andere Körperstruktur beim Einbringen des Implantates kontaktiert, wird dadurch verringert.

Eine einfache konstruktive Ausgestaltung des Instrumentariums wird dadurch ermöglicht, dass das mindestens eine Blockierelement einen mit dem körperseitigen Stützelement oder mit einem dieses umfassenden Träger zusammenwirkenden Anschlag umfasst oder ausbildet.

Es kann insbesondere vorgesehen sein, dass das chirurgische Instrumentarium mehr als nur ein Blockierelement umfasst, wobei mittels jedes Blockierelementes eine Bewegung eines körperseitigen Stützelementes längs der Spreizrichtung verhinderbar ist.

Eine besonders einfache konstruktive Ausgestaltung des Instrumentariums wird ermöglicht, wenn die Fixiereinrichtung das mindestens eine Blockierelement umfasst oder ausbildet.

Es kann auch vorgesehen sein, dass mindestens ein Blockierelement in der Einbringstellung des Implantates an das körperseitige Stützelement oder an einen dieses umfassenden Träger anlegbar und vor dem Überführen des Implantates von der Einbringstellung in die Spreizstellung vom Stützelement oder vom Träger entfernbar ist. Das mindestens eine Blockierelement wird nur solange benötigt, bis ein Operateur das Implantat nach dem Einbringen in den Körper von der Einbringstellung in die Spreizstellung überführen möchte. Bei dieser Ausgestaltung kann das mindestens eine Blockierelement zur Verhinderung der Bewegung des Stützelementes an das Stützelement oder an den Träger angelegt werden. Dies definiert beispielsweise eine Blockierstellung des mindestens einen Blockierelementes. Möchte der Operateur das Implantat in die Spreizstellung überführen, kann er das mindestens eine Blockierelement vom Stützelement oder vom Träger entfernen und in eine dadurch definierte zurückgezogene Stellung überführen.

Von Vorteil ist es, wenn das mindestens eine Blockierelement in einen zwischen einem ein körperseitiges oberes Stützelement umfassenden ersten Träger und einem ein körperseitiges unteres Stützelement umfassenden zweiten Träger, die beim Überführen des Implantates von der Einbringstellung in die Spreizstellung längs der Spreizrichtung relativ zueinander spreizbar sind, gebildeten Zwischenraum einführbar und insbesondere formschlüssig einführbar ist. Dadurch kann auf konstruktive Weise eine zuverlässige Sicherung der Träger gegen eine Spreizbewegung relativ zueinander sichergestellt werden. Damit lässt sich auch eine Bewegung des körperseitigen oberen und des körperseitigen unteren Stützelementes relativ zueinander längs der Spreizrichtung verhindern.

Von Vorteil ist es, wenn das chirurgische Instrumentarium mindestens ein Schutzelement für das Implantat umfasst, denn dadurch kann es gegen Beschädigung gesichert werden.

Vorzugsweise ist das mindestens eine Schutzelement als eine das Implantat einbringseitig zumindest teilweise umgebende Hülle ausgebildet. Dadurch ist die Möglichkeit gegeben, das Implantat insbesondere auf der Einbringseite zu schützen, an welcher ein Operateur typischerweise mit einer Handhabungseinrichtung für das Implantat oder dergleichen angreift. An der Körperseite des Implantates, d.h. demjenigen Bereich des Implantates, der durch den Zwischenwirbelraum hindurch zwischen die Dornfortsätze eingeführt wird, wird das Implantat demgegenüber von der Hülle vorzugsweise nicht umgeben. Dadurch wird zum Einbringen des Implantates in den Zwischenwirbelraum nur wenig Platz beansprucht.

Vorteilhafterweise wird die Hülle ungefähr die Hälfte des Implantates, bezogen auf dessen Erstreckung längs der Spannrichtung, umgeben, um einen zuverlässigen Schutz des Implantates sicherzustellen.

Es kann vorgesehen sein, dass die Hülle die zweite Implantatkomponente zumindest teilweise umgibt. Diese ist dann z.B. einbringseitig angeordnet, während die erste Implantatkomponente körperseitig angeordnet ist und zuerst in den Körper eingebracht wird.

Vorzugsweise umfasst die Fixiereinrichtung das mindestens eine Schutzelement oder bildet dieses aus. Dies ermöglicht es, dem Instrumentarium eine besonders einfache und kompakte Bauform zu verleihen.

Von Vorteil ist es, wenn die Fixiereinrichtung einen im Wesentlichen U-förmigen Querschnitt aufweist. Dadurch kann die Fixiereinrichtung die Gestalt einer Klammer in Form eines großen U verliehen werden. Dies gibt die Möglichkeit, ein Schutzelement für das Implantat zu bilden, um dieses von drei Seiten zu schützen, wenn die Fixiereinrichtung am Implantat angebracht ist.

Zur Ausgestaltung des U-förmigen Querschnittes kann die Fixiereinrichtung zwei die Schenkel des U bildende voneinander beabstandete Segmente umfassen. Die Segmente können an einem ihrer jeweiligen Enden mittels eines Verbindungssegmentes verbunden sein. An ihren jeweiligen freien Enden können das erste und das zweite Segment den vorstehend beschriebenen mindestens einem ersten oberen Anschlag, den mindestens einen zweiten oberen Anschlag sowie ein Knochenanschlagelement umfassen oder ausbilden beziehungsweise den mindestens einen ersten unteren Anschlag, den mindestens einen zweiten unteren Anschlag sowie ein Knochenanschlagelement umfassen oder ausbilden. Am Verbindungselement kann eine Aufnahme für eine und insbesondere die erste Implantatkomponente gebildet sein. In die Aufnahme kann die Implantatkomponente zumindest teilweise eingeführt und so die Fixiereinrichtung am Implantat angebracht werden.

Günstig ist es, wenn die Fixiereinrichtung einstückig ausgebildet ist, denn dies vereinfacht ihre Herstellung.

Vorzugsweise ist die Fixiereinrichtung aus einem Kunststoffmaterial gefertigt, denn dies ermöglicht eine kostengünstige Herstellung der Fixiereinrichtung.

Die Fixiereinrichtung kann insbesondere aus einem verformbaren Material gefertigt sein. Dies gibt beispielsweise die Möglichkeit, die Fixiereinrichtung auf einfachere Weise am Implantat anzubringen oder von diesem zu lösen.

Günstig ist es, wenn die Fixiereinrichtung aus einem elastisch verformbaren Material gefertigt ist. Dies ist insbesondere dann von Vorteil, wenn die Fixiereinrichtung am Implantat anzubringen ist und dabei zu verformen ist. Eine Beschädigung der Fixiereinrichtung kann dadurch verhindert werden.

Es kann vorgesehen sein, dass die Fixiereinrichtung am Implantat vormontiert ist. Das erleichtert die Handhabung des Instrumentariums, da ein Operateur die Fixiereinrichtung nicht vor dem Einbringen am Implantat anbringen muss.

Bevorzugt ist eine am Implantat montierte Fixiereinrichtung von diesem lösbar. Nach dem Überführen des Implantates von der Einbringstellung in die Spreizstellung wird die Fixiereinrichtung nicht weiter benötigt, da das Implantat bestimmungsgemäß an den Dornfortsätzen anliegt. Bei dieser Ausführungsform ist somit die Möglichkeit gegeben, die Fixiereinrichtung nach dem Einsetzen des Implantates aus dem Körper zu entfernen.

Alternativ oder ergänzend kann vorgesehen sein, dass die Fixiereinrichtung aus einem resorbierbaren Material hergestellt ist, und es ist möglich, dass sie nach dem Einsetzen des Implantates in den Zwischenwirbelraum im Körper verbleibt.

Von Vorteil ist es, wenn die Fixiereinrichtung mindestens ein erstes Abziehlagerelement zum Abziehen der Fixiereinrichtung vom Implantat ausbildet, denn dies gibt auf konstruktiv einfache Weise die Möglichkeit, die Fixiereinrichtung vom Implantat zu lösen. An dem ersten Abziehlagerelement kann eine Handhabungseinrichtung des Instrumentariums angreifen, um die Fixiereinrichtung vom Implantat zu lösen.

Bevorzugt umfasst die Fixiereinrichtung mindestens ein in einer quer zur Spannrichtung orientierten Abziehrichtung wirksames Abziehanschlagelement oder bildet dieses aus. Dies erlaubt es, die Fixiereinrichtung quer zur Spannrichtung, ohne die Position der beiden Implantatkomponenten relativ zueinander zu beeinflussen, vom Implantat abzuziehen. Die Abziehrichtung kann insbesondere senkrecht zur Spannrichtung ausgerichtet sein.

Günstig ist es, wenn die Abziehrichtung quer und insbesondere senkrecht zur Spreizrichtung ausgerichtet ist. Dies gibt die Möglichkeit, die Fixiereinrichtung vom Implantat abzuziehen, ohne auf den oberen und/oder den unteren Dornfortsatz unerwünschterweise eine diese auseinander treibende Kraft auszuüben.

Als günstig hat es sich erwiesen, wenn die Fixiereinrichtung ein erstes Spreizlagerelement zum Überführen des Implantates von der Einbringstellung in die Spreizstellung umfasst oder ausbildet. Dies erlaubt es, dem Instrumentarium eine kompaktere Bauform zu verleihen. An dem ersten Spreizlagerelement kann eine längs der Spannrichtung wirksame Spreizkraft zum Spreizen des Implantates angreifen.

Vorzugsweise umfasst die Fixiereinrichtung mindestens ein längs der Spannrichtung wirksames Spreizanschlagelement oder bildet dieses aus. Dadurch ist auf einfache konstruktive Weise die Möglichkeit gegeben, dass die Fixiereinrichtung ein Spreizlagerelement ausbildet. An diesem kann beispielsweise eine Handhabungseinrichtung des Instrumentariums zum Spreizen des Implantates angreifen.

Günstig ist es, wenn das mindestens eine Spreizanschlagelement mit einer von der Fixiereinrichtung gebildeten Aufnahme gekoppelt ist, in der eine und vorzugsweise die erste Implantatkomponente gehalten ist. Das Spreizanschlagelement ist mittels der Aufnahme beispielsweise kraft- und/oder formschlüssig gekoppelt. Dadurch kann eine am Spreizanschlagelement angreifende Spreizkraft auf die Aufnahme und damit auf die darin gehaltene Implantatkomponente, insbesondere die erste Implantatkomponente, übertragen werden. Hierfür umfasst die Aufnahme bevorzugt einen längs der Spannrichtung wirksamen Anschlag für die Implantatkomponente.

Vorzugsweise bildet die vorstehend genannte Hülle ein Koppelelement, dass das erste Spreizlagerelement mit der Aufnahme koppelt, wobei diese und das mindestens eine Spreizlagerelement von der Hülle ausgebildet oder umfasst werden.

Vorteilhafterweise weist das chirurgische Instrumentarium eine Halteeinrichtung auf, die eine Aufnahme für eine Implantatkomponente und insbesondere die zweite Implantatkomponente umfasst, in der diese teilweise gehalten ist. Mittels der Halteeinrichtung kann die Implantatkomponente und insbesondere die zweite Implantatkomponente und damit auch das Implantat gehalten werden.

Alternativ oder ergänzend kann vorgesehen sein, dass das Implantat und vorzugsweise die zweite Implantatkomponente eine Aufnahme umfasst oder ausbildet, in der die Halteeinrichtung gehalten ist.

Bevorzugt bildet die Halteeinrichtung ein zweites Spreizlagerelement zum Überführen des Implantates von der Einbringstellung in die Spreizstellung. Greift an der Halteeinrichtung eine längs der Spannrichtung wirksame Spreizkraft an, kann dadurch das Implantat in die Spreizstellung überführt werden. Die hierfür erforderliche Gegenkraft kann, wie vorstehend erwähnt, an der Fixiereinrichtung angreifen.

Von Vorteil ist es, wenn die Fixiereinrichtung ein Führungselement für die Halteeinrichtung beim Überführen des Implantates von der Einbringstellung in die Spreizstellung ausbildet. Dadurch ist die Möglichkeit gegeben, die Halteeinrichtung in einer klar definierten Richtung relativ zur Fixiereinrichtung zu bewegen und dadurch das Implantat zuverlässig in die Spreizstellung zu überführen.

Grundsätzlich ist es möglich, dass ein Operateur sich allein der Halteeinrichtung bedient, um das Implantat in den Körper einzubringen. Hierfür kann die Halteeinrichtung beispielsweise ein Griffelement umfassen oder ausbilden.

Vorzugsweise umfasst die Halteeinrichtung allerdings eine Verbindungseinrichtung zum Verbinden mit einer ersten Handhabungseinrichtung des chirurgischen Instrumentariums für das Implantat. Dies gibt die Möglichkeit, die Halteeinrichtung mit der ersten Handhabungseinrichtung zu verbinden. Die Halteeinrichtung kann dadurch gewissermaßen ein Zwischenstück ausbilden zwischen der Handhabungseinrichtung und dem Implantat. Dies ist von Vorteil, wenn die Halteeinrichtung ein Wegwerfteil ist, das mit nur einem Implantat zum Einsatz kommt, wohingegen die erste Handhabungseinrichtung ein mehrfach einzusetzendes Werkzeug ist.

Günstig ist es, wenn die Verbindungseinrichtung als Rasteinrichtung ausgebildet ist, denn dadurch ist die Möglichkeit gegeben, die Handhabungseinrichtung auf einfache Weise mit der Halteeinrichtung zu verbinden. Die Rastverbindung zwischen der Halteeinrichtung und der Handhabungseinrichtung kann lösbar sein.

Vorzugsweise weist das Implantat eine ein Verriegelungselement umfassende Verriegelungseinrichtung auf, welches Verriegelungselement in der Spreizstellung des Implantates von einer Freigabestellung, in der die zweite Implantatkomponente relativ zur ersten Implantatkomponente entlang der Spannrichtung beweglich ist, in einer quer zur Spannrichtung ausgerichteten Verriegelungsrichtung in eine Verriegelungsstellung überführbar ist, in der die zweite Implantatkomponente gegen eine der Spannrichtung entgegen gerichtete Bewegung an der ersten Implantatkomponente verriegelt ist. Mittels des Verriegelungselementes kann sichergestellt werden, dass sich die zwei Implantatkomponenten in der Spreizstellung des Implantates nicht relativ zueinander längs der Spannrichtung bewegen. Dadurch behält das Implantat nach dem Einsetzen in den Körper die Spreizstellung bei und kann zum zuverlässigen Abstützen der Dornfortsätze relativ zueinander wirksam werden.

Vorteilhafterweise ist das Verriegelungselement zusammen mit einem von der zweiten Implantatkomponente umfassten und mit dem Verriegelungselement zum Verriegeln des Implantates zusammenwirkenden Fixierelement in der Aufnahme gehalten. Dadurch kann eine zuverlässige Relativpositionierung des Verriegelungselementes und des Fixierelementes zueinander sichergestellt werden. Dies ist sowohl zum Überführen des Verriegelungselementes von der Freigabestellung in die Verriegelungsstellung von Vorteil als auch beim Spreizen des Implantates.

Vorzugsweise umfasst die Halteeinrichtung mindestens ein erstes Kopplungsglied oder bildet dieses aus, das mit mindestens einem vom Verriegelungselement umfassten oder ausgebildeten zweiten Kopplungsglied zum Überführen des Verriegelungselementes von der Freigabestellung in die Verriegelungsstellung zusammenwirkt. Dadurch kann die Halteeinrichtung über die Funktion zum Halten und gegebenenfalls auch zum Spreizen des Implantates hinaus eine weitere Funktion ausüben. Das mindestens eine erste Kopplungsglied koppelt mit dem mindestens einen zweiten Kopplungsglied des Verriegelungselementes, so dass dieses mittels der Halteeinrichtung in die Verriegelungsstellung überführt werden kann. Dies erleichtert die Handhabung des Instrumentariums.

Bei einer einfachen konstruktiven Ausgestaltung des Instrumentariums ist das mindestens eine erste Kopplungsglied als längs der Verriegelungsrichtung wirksamer Mitnehmeranschlag für das Verriegelungselement ausgebildet. Das mindestens eine zweite Kopplungsglied ist in entsprechender Weise als ein Anschlag ausgebildet, der mit dem Mitnehmeranschlag zusammenwirkt. Wird die Halteeinrichtung mit einer in der Verriegelungsrichtung wirkenden Kraft beaufschlagt, koppeln die Kopplungsglieder miteinander, so dass das Verriegelungselement in die Verriegelungsstellung überführt werden kann.

Günstig ist es, wenn die Halteeinrichtung in der Verriegelungsstellung des Verriegelungselementes vom Implantat in der Verriegelungsrichtung abziehbar ist. Wenn das Implantat verriegelt ist, wird die Halteeinrichtung nicht mehr benötigt. Sie kann bei dieser Ausführungsform vom Implantat in der Verriegelungsrichtung abgezogen werden, beispielsweise indem sie im Anschluss an das Verriegeln des Implantates weiterhin mit einer in der Verriegelungsrichtung wirkenden Kraft beaufschlagt wird.

Günstigerweise umfasst die Aufnahme eine Öffnung, durch die hindurch das Fixierelement und das Verriegelungselement in der Verriegelungsstellung des Verriegelungselementes aus der Aufnahme entfernbar sind. Die Öffnung definiert beispielsweise eine quer und insbesondere senkrecht zur Verriegelungsrichtung ausgerichtete Querschnittsfläche, durch die hindurch das Fixierelement und das Verriegelungselement aus der Aufnahme entfernt werden können. Dies erlaubt es, die Halteeinrichtung vom Implantat abzuziehen, und das Fixierelement und das Verriegelungselement verbleiben am verriegelten Implantat.

Das mindestens eine Kopplungsglied der Halteeinrichtung ist günstigerweise an einem Rand der Öffnung angeordnet.

Bevorzugt weist die Verriegelungsrichtung eine parallele Komponente zu einer Abziehrichtung für die Fixiereinrichtung vom Implantat auf, und insbesondere ist die Verriegelungsrichtung parallel zur Abziehrichtung ausgerichtet. Dies erlaubt es, sowohl die Fixiereinrichtung als auch die Halteeinrichtung in derselben Richtung - günstigerweise senkrecht zur Spannrichtung und senkrecht zur Spreizrichtung - vom Implantat abzuziehen.

Von Vorteil ist es, wenn die Halteeinrichtung am Implantat vormontiert ist, denn dies erleichtert einem Operateur die Handhabung des Instrumentariums.

Es kann auch vorgesehen sein, dass die Halteeinrichtung am Implantat anbringbar ist. Hierzu kann beispielsweise vorgesehen sein, dass die vorstehend erwähnte Aufnahme erweiterbar ist, so dass das Fixierelement und das Verriegelungselement in die Aufnahme eingeführt werden können, um das Implantat an der Halteeinrichtung anzubringen.

Die Halteinrichtung ist vorzugsweise Bestandteil des Implantates und wird von diesem umfasst, insbesondere wenn sie am Implantat vormontiert ist.

Es ist allerdings auch möglich, dass die Halteeinrichtung Bestandteil der nachfolgend beschriebenen ersten Handhabungseinrichtung ist und von dieser umfasst wird, insbesondere dann, wenn sie am Implantat anbringbar ist.

Zur Erzielung einer einfachen Konstruktion der Halteeinrichtung ist es günstig, wenn sie einstückig ausgebildet ist.

Es wurde bereits erwähnt, dass das Instrumentarium eine erste Handhabungseinrichtung aufweisen kann. Insbesondere ist es günstig, wenn das chirurgische Instrumentarium eine ein Griffelement umfassende erste Handhabungseinrichtung zum Halten des Implantates umfasst. Am Griffelement kann der Operateur auf benutzerfreundliche Weise angreifen, um das Implantat in den Körper einzubringen.

Ein chirurgisches Instrumentarium der eingangs genannten Art mit einer Handhabungseinrichtung zum Halten eines Implantates ermöglicht, wenn die Handhabungseinrichtung ein Griffelement aufweist, einem Operateur eine benutzerfreundliche Handhabung der Handhabungseinrichtung und damit des Implantates, indem er am Griffelement angreift.

Grundsätzlich kann vorgesehen sein, dass das Implantat unmittelbar mittels der ersten Handhabungseinrichtung gehalten werden kann.

Es ist allerdings auch möglich, dass die erste Handhabungseinrichtung eine Verbindungseinrichtung zum Verbinden mit einer Halteeinrichtung für das Implantat umfasst. Hierbei handelt es sich günstigerweise um die vorstehend beschriebene Halteeinrichtung für das Implantat, an welcher seinerseits das Implantat und insbesondere die zweite Implantatkomponente gehalten ist.

Die Verbindungseinrichtung ist günstigerweise als Rasteinrichtung ausgebildet, speziell als lösbare Rasteinrichtung, um einem Operateur das Verbinden der ersten Handhabungseinrichtung mit der Halteeinrichtung zu erleichtern sowie gegebenenfalls das Lösen zu ermöglichen.

Bei einer einfachen konstruktiven Ausgestaltung ist die erste Handhabungseinrichtung als eine Längserstreckung aufweisender Griffarm ausgebildet. Der Griffarm mit Längserstreckung ermöglicht einem Operateur ein benutzerfreundliches Einbringen des Implantates in den Zwischenwirbelraum.

Vorteilhafterweise umfasst das chirurgische Instrumentarium eine zweite Handhabungseinrichtung für das Implantat, die relativ zur ersten Handhabungseinrichtung beweglich ausgebildet ist und mit dieser zum Überführen des Implantates von der Einbringstellung in die Spreizstellung zusammenwirkt. Dies bietet dem Operateur die Möglichkeit, durch Angreifen an der ersten und an der zweiten Handhabungseinrichtung das Implantat zu spreizen.

Vorzugsweise ist die zweite Handhabungseinrichtung relativ zur ersten Handhabungseinrichtung von einer ersten Stellung in eine zweite Stellung überführbar, wobei ein Ende der zweiten Handhabungseinrichtung längs der Spannrichtung relativ zu einem das Implantat haltende Ende der ersten Handhabungseinrichtung in der ersten Stellung einen anderen Abstand aufweist als in der zweiten Stellung. Greift das Ende der zweiten Handhabungseinrichtung beim Überführen von der ersten Stellung in die zweite Stellung am Implantat an, kann dadurch auf das Implantat eine Spreizkraft eingeleitet werden, deren erforderliche Gegenkraft mit der ersten Handhabungseinrichtung auf das Implantat eingeleitet werden kann.

Zur Erzielung einer zuverlässigen Relativbewegung der zweiten Handhabungseinrichtung und der ersten Handhabungseinrichtung zueinander ist es von Vorteil, wenn die zweite Handhabungseinrichtung an der ersten Handhabungseinrichtung beweglich gelagert ist.

Als besonders günstig hat es sich in der Praxis erwiesen, wenn die zweite Handhabungseinrichtung an der ersten Handhabungseinrichtung um eine quer zur Spannrichtung orientierte Schwenkachse schwenkbar gelagert ist. Dies erlaubt es, dem Instrumentarium eine einfache Konstruktion zu verleihen.

Eine besonders einfache Konstruktion des Instrumentariums ist erzielbar, wenn die zweite Handhabungseinrichtung als Schwenkhebel ausgebildet ist.

Von Vorteil ist es, wenn die zweite Handhabungseinrichtung mindestens ein zweites Spreizanschlagelement umfasst oder ausbildet, das beim Überführen des Implantates von der Einbringstellung in die Spreizstellung mit mindestens einem, vorzugsweise von der Fixiereinrichtung umfassten oder ausgebildeten, ersten Spreizanschlagelement zusammenwirkt. Das mindestens eine zweite Spreizanschlagelement, das günstigerweise endseitig an der zweiten Handhabungseinrichtung angeordnet ist, kann an das mindestens eine erste Spreizanschlagelement angelegt werden. Beim Überführen der zweiten Handhabungseinrichtung von der ersten Stellung in die zweite Stellung kann dadurch eine längs der Spannrichtung wirksame Kraft auf das Implantat und insbesondere die Fixiereinrichtung ausgeübt werden.

Günstig ist es, wenn die zweite Handhabungseinrichtung mindestens ein zweites Abziehanschlagelement umfasst oder ausbildet, das zum Abziehen der Fixiereinrichtung vom Implantat mit mindestens einem von der Fixiereinrichtung umfassten oder ausgebildeten ersten Abziehanschlagelement zusammenwirkt. Dadurch kann eine längs der Abziehrichtung wirksame Kraft von der zweiten Handhabungseinrichtung auf die Fixiereinrichtung ausgeübt werden und dadurch diese vom Implantat abgezogen werden. Eine hierfür erforderliche Gegenkraft kann beispielsweise von der nachstehend beschriebenen dritten Handhabungseinrichtung des Instrumentariums auf das Implantat ausgeübt werden.

Das mindestens eine zweite Abziehanschlagelement ist beispielsweise endseitig an der zweiten Handhabungseinrichtung angeordnet.

Alternativ oder ergänzend kann vorgesehen sein, dass das mindestens eine Abziehanschlagelement nicht von der zweiten Handhabungseinrichtung umfasst oder ausgebildet wird. Das Instrumentarium kann beispielsweise hierfür eine gesonderte Abzieheinrichtung mit dem mindestens einen zweiten Abziehanschlagelement umfassen, die separat von der zweiten Handhabungseinrichtung ausgebildet ist.

Vorzugsweise umfasst das chirurgische Instrumentarium eine Sperreinrichtung mit einem von der ersten Handhabungseinrichtung umfassten ersten Sperrelement und einem von der zweiten Handhabungseinrichtung umfassten zweiten Sperrelement, die relativ zueinander von einer Sperrstellung, in der das erste Sperrelement und das zweite Sperrelement zum Sperren einer Bewegung der zweiten Handhabungseinrichtung relativ zur ersten Handhabungseinrichtung zusammenwirken, in eine Lösestellung, in der das erste Sperrelement und das zweite Sperrelement nicht zusammenwirken und die zweite Handhabungseinrichtung relativ zur ersten Handhabungseinrichtung beweglich ist, und umgekehrt, überführbar ist. Mittels der Sperreinrichtung kann sichergestellt werden, dass sich die erste und die zweite Handhabungseinrichtung nicht unerwünschterweise relativ zueinander bewegen. Wird das Implantat in den Zwischenwirbelraum eingebracht, nehmen das erste und das zweite Sperrelement günstigerweise die Sperrstellung ein. Dadurch kann vermieden werden, dass sich das Implantat unerwünschterweise aufspreizt. Erst durch eine bewusste Handlung des Operateurs, der das erste und das zweite Sperrelement in die Lösestellung überführt, kann die zweite Handhabungseinrichtung relativ zur ersten Handhabungseinrichtung bewegt und dadurch das Implantat gespreizt, d.h. von der Einbringstellung in die Spreizstellung überführt werden.

Vorzugsweise sind das erste Sperrelement und das zweite Sperrelement in der Lösestellung relativ zueinander bezüglich ihrer Sperrstellung vorgespannt. Dadurch ist die Möglichkeit gegeben, dass die Sperrstellung gewissermaßen eine Grund- oder Ruhestellung der Sperrelemente bildet. Die Sperrstellung können die Sperrelemente immer dann einnehmen, wenn der Operateur sie nicht bewusst in ihre Lösestellung überführt oder in dieser hält. Beim Überführen des Implantates von der Einbringstellung in die Spreizstellung ist dies besonders vorteilhaft, denn das Implantat wird typischerweise in mehreren kleinen Schritten gespreizt. Der Operateur kann dadurch sukzessive das Implantat spreizen, indem er die Sperrelemente in der Lösestellung hält, anschließend aber so weit entgegen der Vorspannung nachgibt, dass sie ihre Sperrstellung einnehmen. Das Implantat ist dadurch bei jedem Spreizschritt gegen ein Auseinanderspreizen gesichert. Dies erleichtert die Handhabung des Instrumentariums.

Bei einer konstruktiv einfachen Ausgestaltung der Sperreinrichtung ist es von Vorteil, wenn sie ein Rastgesperre ausbildet.

Insbesondere kann vorgesehen sein, dass ein Sperrelement, beispielsweise das erste Sperrelement, als Raststange ausgebildet ist und dass ein Sperrelement, beispielsweise das zweite Sperrelement, als Rasthebel ausgebildet ist. Die Raststange kann mit dem Rasthebel in einer Vielzahl von Raststellungen miteinander verrasten. Dadurch ist die Möglichkeit gegeben, dass die zweite Handhabungseinrichtung relativ zur ersten Handhabungseinrichtung in einer Vielzahl von Stellungen gesichert werden kann, die jeweils mit einem unterschiedlich weiten Aufspreizen des Implantates verknüpft sind. Der Rasthebel kann zugleich ein Betätigungshebel der zweiten Handhabungseinrichtung sein.

Zur Vorspannung der Sperrelemente relativ zueinander umfasst die Sperreinrichtung günstigerweise mindestens ein elastisches Element, beispielsweise in Form einer Feder. Diese kann etwa das zweite Sperrelement mit einer Sperrkaft beaufschlagen, die der Operateur überwinden muss, um die Sperrelemente von der Sperrstellung in die Lösestellung zu überführen.

Von Vorteil ist es, wenn das chirurgische Instrumentarium eine Anzeigeeinrichtung umfasst zum Anzeigen der Relativposition der ersten Handhabungseinrichtung und der zweiten Handhabungseinrichtung. Anhand der Anzeigeeinrichtung kann der Operateur ermitteln, wie weit das Implantat aufgespreizt ist.

Es kann vorgesehen sein, dass die Anzeigeeinrichtung so kalibriert ist, dass der absolute Abstand der oberen und der unteren Stützfläche voneinander für einen Operateur erkennbar ist.

Es hat sich in der Praxis als günstig erwiesen, wenn die Anzeigeeinrichtung an der Sperreinrichtung angeordnet ist. Dadurch ist sie für einen Operateur gut zu erkennen.

Bevorzugt umfasst das chirurgische Instrumentarium eine dritte Handhabungseinrichtung für das Implantat, die relativ zur ersten Handhabungseinrichtung beweglich ausgebildet ist und die mit dem Implantat zum Abziehen der Fixiereinrichtung und/oder einer Halteeinrichtung oder der ersten Handhabungseinrichtung vom Implantat in dessen Spreizstellung zusammenwirkt. Wie bereits erwähnt, kann mittels der dritten Handhabungseinrichtung beispielsweise eine Kraft auf das Implantat ausgeübt werden, die als Gegenkraft zu einer Abziehkraft zum Abziehen der Fixiereinrichtung vom Implantat wirkt. In entsprechender Weise kann die Gegenkraft zum Abziehen der Halteeinrichtung oder, gegebenenfalls, einer das Implantat unmittelbar haltenden ersten Handhabungseinrichtung vom Implantat wirksam werden. Dies ermöglicht eine einfache Handhabung des Instrumentariums.

Vorteilhafterweise ist die dritte Handhabungseinrichtung relativ zur ersten Handhabungseinrichtung von einer zurückgezogenen Stellung in eine Anlegestellung überführbar, wobei mindestens ein von der dritten Handhabungseinrichtung umfasstes oder ausgebildetes Gegenanschlagelement in der zurückgezogenen Stellung einen Abstand vom Implantat aufweist und in der Anlegestellung an diesem anliegt. Dies gibt die Möglichkeit, die dritte Handhabungseinrichtung nach dem Spreizen des Implantates relativ zur ersten Handhabungseinrichtung, an der das Implantat gehalten ist, in Richtung auf das Implantat von der zurückgezogenen Stellung in die Anlegestellung zu überführen. In der Anlegestellung kann das mindestens eine Gegenanschlagelement am Implantat anliegen. Dadurch kann die vorstehend erwähnte Gegenkraft zum Abziehen der Fixiereinrichtung und/oder der Halteeinrichtung oder, gegebenenfalls, der ersten Halteeinrichtung vom Implantat ausgeübt werden.

Das mindestens eine Gegenanschlagelement kann insbesondere endseitig an der dritten Handhabungseinrichtung angeordnet sein.

Vorzugsweise ist die dritte Handhabungseinrichtung an der ersten Handhabungseinrichtung beweglich gelagert. Dies ermöglicht ein zuverlässiges Überführen der dritten Handhabungseinrichtung von der zurückgezogenen Stellung in die Anlegestellung.

Das Überführen der dritten Handhabungseinrichtung hat sich in der Praxis als besonders zuverlässig erwiesen, wenn die dritte Handhabungseinrichtung an der ersten Handhabungseinrichtung verschieblich gelagert ist entlang einer parallel zur Abziehrichtung der Fixiereinrichtung vom Implantat und/oder parallel zur Verriegelungsrichtung des Verriegelungselementes ausgerichteten Richtung.

Bei einer besonders einfachen konstruktiven Ausgestaltung ist die dritte Handhabungseinrichtung als Schieber ausgebildet.

Von Vorteil ist es, wenn die dritte Handhabungseinrichtung ein erstes Segment mit einem Gegenanschlagelement sowie ein zweites Segment mit einem Gegenanschlagelement umfasst und wenn das Gegenanschlagelement des ersten Segmentes längs der Spannrichtung einen Abstand zum Gegenanschlagelement des zweiten Segmentes aufweist. Das erste Segment und das zweite Segment können dadurch in der Anlegestellung jeweils mit einem Gegenanschlagelement diesseits und jenseits eines Verbindungsbereiches der ersten Handhabungseinrichtung mit dem Implantat an dieses angelegt werden. Der Verbindungsbereich ist, bezogen auf die Spannrichtung, zwischen den Gegenanschlagelementen angeordnet. Greift an der ersten Handhabungseinrichtung eine vom Implantat weg weisende Zugkraft an, kann die erste Handhabungseinrichtung oder die Halteeinrichtung dadurch auf zuverlässige Weise ohne zu verkeilen oder zu verkanten vom Implantat gelöst werden.

Insbesondere können die Gegenanschlagelemente des ersten Segmentes und des zweiten Segmentes, bezogen auf die Spannrichtung, in der Anlegestellung seitlich neben der Halteeinrichtung für das Implantat an das Implantat angelegt werden. Es hat sich in der Praxis gezeigt, dass die Halteeinrichtung dadurch besonders zuverlässig vom Implantat abgezogen werden kann.

Ist die dritte Handhabungseinrichtung wie vorstehend beschrieben als Schieber ausgebildet, kann sie die erste Handhabungseinrichtung beispielsweise umgreifen, wobei das erste und das zweite Segment an einander abgewandten Seiten der ersten Handhabungseinrichtung und/oder der Halteeinrichtung angeordnet sind.

Von Vorteil ist es, wenn die dritte Handhabungseinrichtung mindestens ein Blockierelement ausbildet zum Verhindern einer Bewegung eines körperseitigen Stützelementes längs der Spreizrichtung in der Einbringstellung des Implantates, welches mindestens eine Blockierelement in der Einbringstellung am körperseitigen Stützelement oder an einem es umfassenden Träger anlegbar ist, wobei die dritte Handhabungseinrichtung eine Blockierstellung einnimmt. Dadurch kann die dritte Handhabungseinrichtung, wie vorstehend beschrieben, vor dem Aufspreizen des Implantates in einer Blockierstellung zum Blockieren des körperseitigen Stützelementes eingesetzt werden und somit eine zusätzliche Funktion ausüben.

Das mindestens eine Blockierelement kann beispielsweise in der Blockierstellung in einen Zwischenraum zwischen zwei Träger eingreifen, die jeweils ein körperseitiges Stützelement umfassen und die beim Überführen des Implantates von der Einbringstellung in die Spreizstellung entlang der Spreizrichtung relativ zueinander spreizbar sind, um die Bewegung der Stützelemente relativ zueinander zu blockieren.

Günstig ist es, wenn die dritte Handhabungseinrichtung in der Einbringstellung des Implantates von der zurückgezogenen Stellung in die Blockierstellung und umgekehrt überführbar ist. Dies ermöglicht es, vor dem Einbringen des Implantates in den Körper das mindestens eine Blockierelement an das körperseitige Stützelement oder dessen Träger anzulegen, indem die dritte Handhabungseinrichtung von der zurückgezogenen Stellung in die Blockierstellung überführt wird. Vor dem Spreizen des Implantates kann die dritte Handhabungseinrichtung wieder in die zurückgezogene Stellung überführt werden und das mindestens eine Blockierelement in Abstand zum Implantat gebracht werden.

Es kann vorgesehen sein, dass das mindestens eine Blockierelement an einem Ende der dritten Handhabungseinrichtung und insbesondere dessen zweiten Segmentes, gebildet ist oder davon umfasst wird.

Es kann ferner vorgesehen sein, dass das erste Segment relativ zum zweiten Segment beweglich ausgebildet ist und dass das erste Segment und das zweite Segment zum Überführen der dritten Handhabungseinrichtung von der zurückgezogenen Stellung in die Blockierstellung und umgekehrt und/oder von der zurückgezogenen Stellung in die Anlegestellung jeweils mittels paarweise zusammenwirkender und vom ersten Segment und vom zweiten Segment umfassten oder ausgebildeter Koppelelemente koppeln. Die Koppelelemente bilden hierbei beispielsweise zusammenwirkende Anschlagelemente, insbesondere Mitnehmeranschlagelemente.

Weiter kann vorgesehen sein, dass unter der Wirkung der Anlage des zweiten Segmentes am Implantat in der Anlagestellung die die Kopplung zwischen dem ersten Segment und dem zweiten Segment bewirkenden Koppelelemente zum Überführen der dritten Handhabungseinrichtung von der zurückgezogenen Stellung in die Blockierstellung voneinander entkoppeln. Dies erlaubt es, eine Relativbewegung des ersten Segmentes und des zweiten Segmentes zu erzielen, wenn das zweite Segment in die Anlagestellung gelangt. Dann kann das erste Segment solange relativ zum zweiten Segment bewegt werden, bis die Segmente erneut unter der Wirkung zusammenwirkender Koppelelemente koppeln, um über beide Segmente eine Kraft auf das Implantat einzuleiten.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Ansicht eines Implantates, einer Fixiereinrichtung und einer Halteeinrichtung einer ersten bevorzugten Ausführungsform eines erfindungsgemäßen chirurgischen Instrumentariums in einer Explosionsdarstellung;
- Figur 2A:: eine Seitenansicht des Implantates und der Halteeinrichtung aus Figur 1 im montierten Zustand, wobei das Implantat eine Grundstellung einnimmt;
- Figur 2B:: eine Seitenansicht des Implantates, der Fixiereinrichtung und der Halteeinrichtung aus Figur 1 im montierten Zustand, wobei das Implantat eine Einbringstellung einnimmt;
- Figur 3:: eine perspektivische Darstellung des Implantates und der Halteeinrichtung aus Figur 1 im montierten Zustand;
- Figur 4:: eine perspektivische Darstellung des chirurgischen Instrumentariums gemäß der ersten Ausführungsform mit dem Implantat, der Fixiereinrichtung und der Halteeinrichtung aus Figur 1 im montierten Zustand und mit einer ersten, einer zweiten und einer dritten Handhabungseinrichtung in Explosionsdarstellung;
- Figur 5:: das Instrumentarium aus Figur 5 beim Einbringen des Implantates in einen Raum zwischen zwei Dornfortsätze, wobei das Implantat die Einbringstellung einnimmt;
- Figur 6:: das Instrumentarium aus Figur 4 nach dem Überführen des Implantates von der Einbringstellung in eine Spreizstellung, in der es die Dornfortsätze gegeneinander abstützt;
- Figur 7:: eine vergrößerte Darstellung von Detail A in Figur 6, teilweise geschnitten;
- Figur 8A:: eine Detailansicht einer Sperreinrichtung des Instrumentariums längs der Linie 8-8 in Figur 6, wobei die Sperreinrichtung eine Lösestellung einnimmt;
- Figur 8B:: die Sperreinrichtung aus Figur 8A in einer Sperrstellung;
- Figur 9:: eine perspektivische Teilansicht, teilweise geschnitten, eines den Dornfortsätzen zugewandten Endbereiches des Instrumentariums, wobei das Implantat entsprechend Figur 6 die Spreizstellung einnimmt;
- Figur 10:: eine Schnittansicht längs der Linie 10-10 in Figur 9;
- Figur 11:: eine Schnittansicht längs der Linie 11-11 in Figur 9, wobei ein Verriegelungselement des Instrumentariums eine Freigabestellung einnimmt;
- Figur 12:: eine Darstellung entsprechend Figur 11 mit dem Verriegelungselement in der Verriegelungsstellung und zu Beginn des Lösens der Halteeinrichtung und der Fixiereinrichtung vom Implantat;
- Figur 13:: eine perspektivische Teildarstellung des Instrumentariums mit dem zwischen die Dornfortsätze eingesetztem Implantat, von dem die Fixiereinrichtung und die Halteeinrichtung gelöst wurden;
- Figur 14:: eine perspektivische Ansicht eines Implantates, einer Fixiereinrichtung und einer Halteeinrichtung einer zweiten bevorzugten Ausführungsform eines erfindungsgemäßen chirurgischen Instrumentariums in einer Explosionsdarstellung;
- Figur 15A:: eine Seitenansicht des Implantates und der Halteeinrichtung aus Figur 14 im montierten Zustand, wobei das Implantat eine Grundstellung einnimmt;
- Figur 15B:: eine Seitenansicht des Implantates, der Fixiereinrichtung und der Halteeinrichtung aus Figur 14 im montierten Zustand, wobei das Implantat eine Einbringstellung einnimmt;
- Figur 16:: eine perspektivische Darstellung des chirurgischen Instrumentariums gemäß der zweiten Ausführungsform mit dem Implantat, der Fixiereinrichtung und der Halteeinrichtung aus Figur 14 im montierten Zustand und mit einer ersten, einer zweiten und einer dritten Handhabungseinrichtung in Explosionsdarstellung;
- Figur 17:: das Instrumentarium aus Figur 16 in einer perspektivischen Teildarstellung, ohne die zweite Handhabungseinrichtung;
- Figur 18:: das Instrumentarium aus Figur 16 beim Einbringen des Implantates in einen Raum zwischen zwei Dornfortsätze, wobei das Implantat die Einbringstellung einnimmt;
- Figur 19:: eine vergrößerte Darstellung von Detail B in Figur 18;
- Figur 20:: das Instrumentarium aus Figur 18 nach dem Überführen des Implantates von der Einbringstellung in die Spreizstellung und
- Figur 21:: eine Schnittansicht längs der Linie 21-21 in Figur 20.

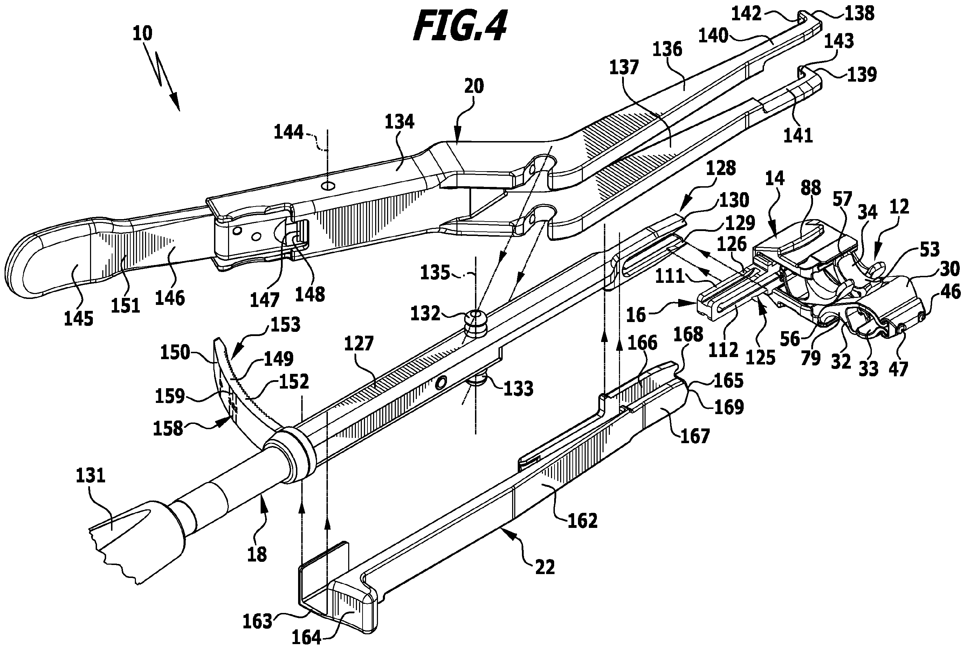

Eine in Figur 4 teilweise in Explosionsdarstellung gezeigte erste bevorzugte Ausführungsform eines erfindungsgemäßen chirurgischen Instrumentariums 10 umfasst ein Implantat 12, eine Fixiereinrichtung 14, eine Halteeinrichtung 16 sowie ferner eine erste, eine zweite sowie eine dritte Handhabungseinrichtung 18, 20 bzw. 22 für das Implantat 12, die Fixiereinrichtung 14 und die Halteeinrichtung 16.

Das Implantat 12 ist ein Zwischenwirbelimplantat zur gegenseitigen Abstützung eines oberen Dornfortsatzes 24 eines ersten Wirbelkörpers 25 und eines unteren Dornfortsatzes 26 eines unteren Wirbelkörpers 27. Es gehört zu einer Klasse von in der DE 20 2008 009 344 U1 beschriebenen Zwischenwirbelimplantaten.

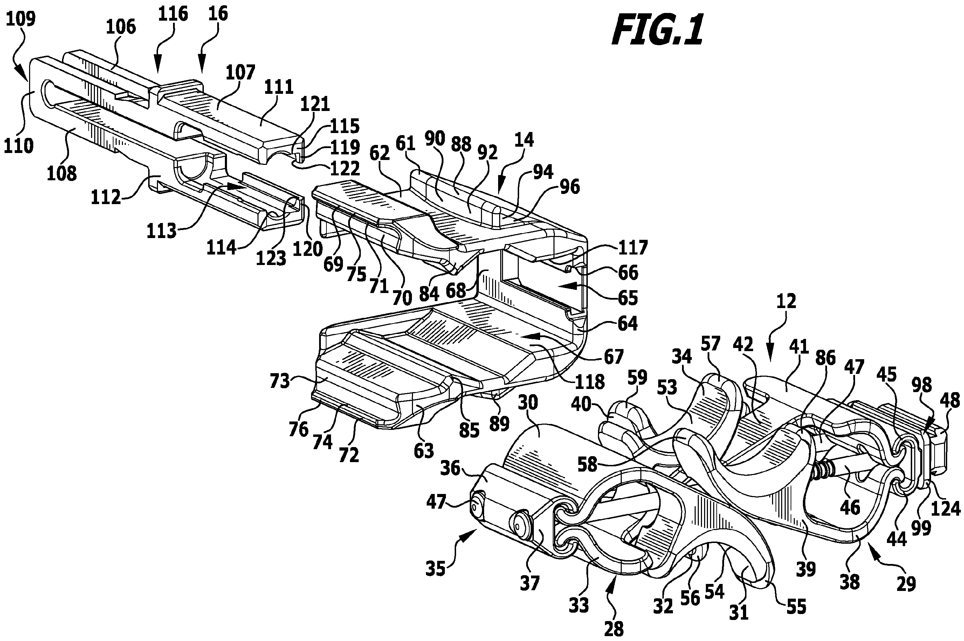

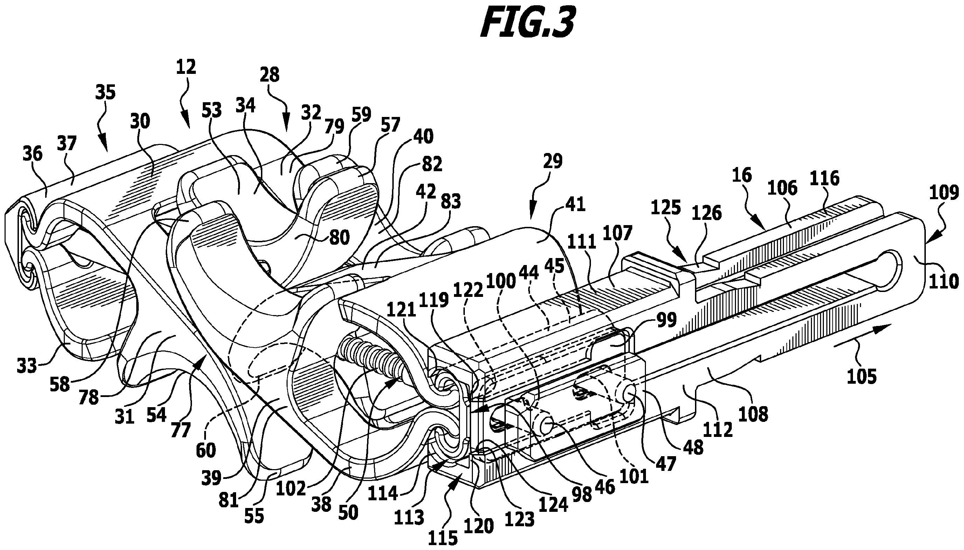

Wie insbesondere aus den Figuren 1 und 3 deutlich wird, umfasst das Implantat 12 eine erste Implantatkomponente 28 sowie eine zu dieser im Wesentlichen identisch ausgebildete, zweite Implantatkomponente 29. Die erste Implantatkomponente 28 umfasst ein erstes Stützsegment 30, das zwei Stützarme 31 und 32 umfasst, sowie ein zweites Stützsegment 33, das einen zwischen dem ersten Stützarm 31 und dem zweiten Stützarm 32 angeordneten Stützarm 34 umfasst. Die Stützsegmente 30 und 33 sind jeweils aus einem elastisch verformbaren Material gebildet und an einer ersten Endseite 35 des Implantates 12 mittels eines Haltegliedes 36 in Form einer C-förmigen Klammer 37 zusammengehalten.

In entsprechender Weise umfasst die zweite Implantatkomponente 29 ein erstes Stützsegment 38 mit zwei voneinander beabstandeten Stützarmen 39 und 40 sowie ein zweites Stützsegment 41 mit einem zwischen den Stützarmen 39 und 40 angeordneten Stützarm 42. Die Stützsegmente 38 und 41 sind ebenfalls aus einem elastisch verformbaren Material gebildet, und sie sind nahe einer der Endseite 35 gegenüberliegenden Endseite 43 des Implantates 12 mittels eines Haltegliedes 44 in Form einer C-förmigen Klammer 45 zusammengehalten.

Die Implantatkomponenten 28 und 29 sind relativ zueinander spiegelsymmetrisch bezüglich eines in der Zeichnung nicht dargestellten Symmetriezentrums ausgebildet. Dies hat zur Folge, dass die Stützarme 31 und 39, die Stützarme 32 und 40 sowie die Stützarme 34 und 42 aneinander anliegen können.

Zwei an der Klammer 37 festgelegte Spannelemente 46 und 47 reichen von der Endseite 35 bis zur Endseite 43. Dabei durchgreifen sie das Implantat 12 entlang dessen Längserstreckung. An der Endseite 43 sind sie mittels eines Verbindungsgliedes 48 verbunden. Die Erstreckungsrichtung der Spannelemente 46 und 47 definiert eine Spannrichtung 49 des Implantates 12, und gemeinsam bilden die Spannelemente 46 und 47 einen Zuganker 50 des Implantates 12.

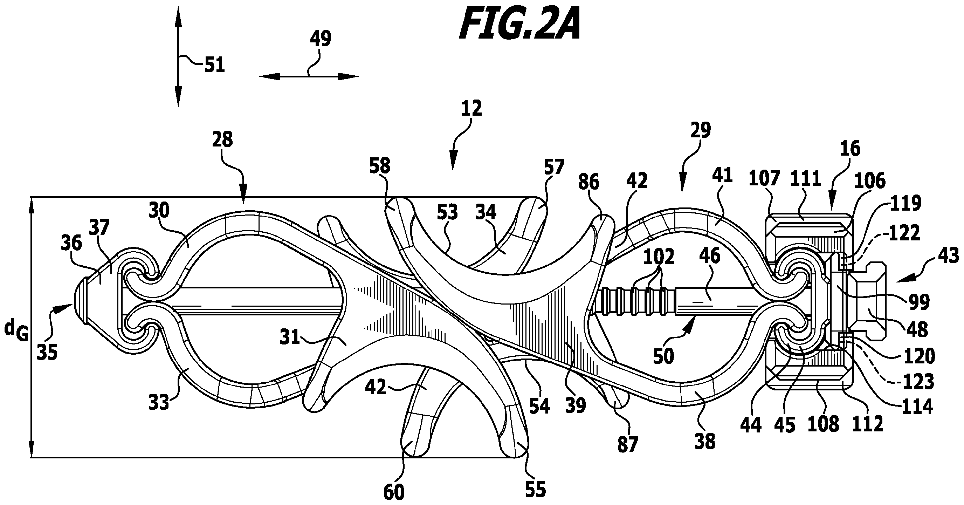

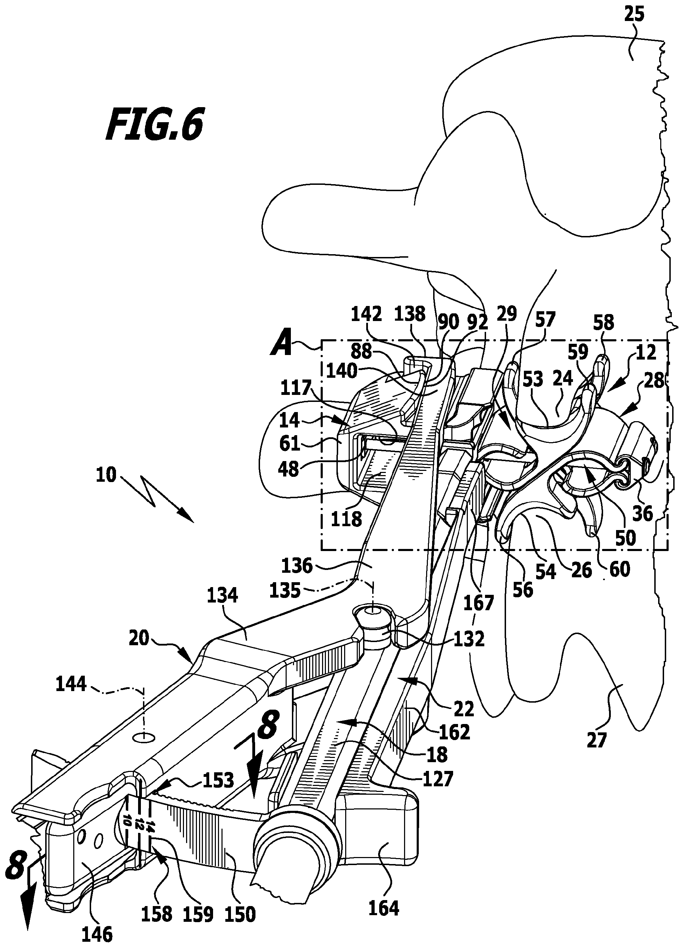

Grundsätzlich ist es möglich, das Implantat 12 ausgehend von einer in Figur 2A dargestellten Grundstellung, in der die Klammer 45 nahe dem Verbindungsglied 48 angeordnet ist, durch Kraftbeaufschlagung beispielsweise der Klammer 45 entlang der Spannrichtung 49 in eine beispielsweise in den Figuren 6, 7 und 13 dargestellte Spreizstellung zu überführen. Dies erfolgt durch Verschieben der zweiten Implantatkomponente 29 relativ zur ersten Implantatkomponente 28 entlang des Zugankers 50. Dabei bilden die Stützarme 31 und 39 jeweils Aufgleitelemente füreinander, die Stützarme 32 und 40 bilden Aufgleitelemente füreinander, und die Stützarme 34 und 42 bilden Aufgleitelemente füreinander. Dies bedeutet, dass sich die Stützarme 31 und 39 gegeneinander abstützen und aneinander entlang gleiten. In entsprechender Weise stützen sich die Stützarme 32 und 40 sowie die Stützarme 34 und 42 gegeneinander ab und gleiten aneinander entlang. Dadurch spreizt sich das Implantat 12 in einer senkrecht zur Spannrichtung 49 ausgerichteten Spreizrichtung 51.

In der Spreizstellung des Implantates 12 weisen eine von den Stützarmen 39, 40 und 34 gebildete obere Stützfläche 53 für den oberen Dornfortsatz 24 sowie eine von den Stützarmen 31, 32 und 42 gebildete untere Stützfläche 54 für den unteren Dornfortsatz 26 voneinander einen größeren Abstand auf als in der Grundstellung. Dies gibt die Möglichkeit, die Dornfortsätze 24 und 26 gegeneinander abzustützen.

Die Stützarme 31 und 32 umfassen an ihren freien Enden jeweils ein Stützelement 55 bzw. 56, die aus nachfolgend noch ersichtlichen Gründen als "einbringseitige" untere Stützelemente bezeichnet werden. Am freien Ende umfasst der Stützarm 34 ein einbringseitiges oberes Stützelement 57. In entsprechender Weise umfassen die Stützarme 39 und 40 an ihren freien Enden jeweils ein Stützelement 58 bzw. 59, die nachfolgend als "körperseitige" obere Stützelemente bezeichnet werden. Der Stützarm 42 umfasst an seinem freien Ende ein körperseitiges unteres Stützelement 60. Die Stützarme 31, 32, 34, 39, 40 und 42 bilden somit Träger der Stützelemente 55, 56, 57, 58, 59 beziehungsweise 60.

In der Spreizstellung des Implantates 12 können die einbringseitigen Stützelemente 55, 56 und 57 die Dornfortsätze 26 bzw. 24 lateral abstützen und hierfür beispielsweise an diesen anliegen, und zwar an denjenigen Seiten der Dornfortsätze 24 und 26, die einem Zugang in den menschlichen Körper zugewandt sind, durch welchen das Implantat 12 in den Körper eingebracht wird (Figuren 6, 7 und 13).

In entsprechender Weise können in der Spreizstellung des Implantates 12 die körperseitigen oberen Stützelemente 58, 59 und 60 die Dornfortsätze 24 bzw. 26 lateral abstützen und hierfür beispielsweise an diesen anliegen, und zwar körperseitig, d.h. jeweils an denjenigen Seiten der Dornfortsätze 24 und 26, die einem Zugang für das Implantat 12 abgewandt sind.

In der Grundstellung des Implantates (Figur 2A) weist das einbringseitige obere Stützelement 57 von den einbringseitigen unteren Stützelementen 55 und 56 einen Abstand d_{G} längs der Spreizrichtung 51 auf, der identisch ist zum Abstand des körperseitigen unteren Stützelementes 60 von den körperseitigen oberen Stützelementen 58 und 59 längs der Spreizrichtung 51. Dieser Abstand d_{G} definiert die Einbringhöhe des Implantates 12 in dessen Grundstellung, und er muss insbesondere kleiner sein als der Abstand der Dornfortsätze 24 und 26 voneinander, damit das Implantat 12 in den Zwischenwirbelraum eingebracht werden kann.

Um das Implantat 12 ausgehend von der Grundstellung in eine zum Einbringen in den Körper bevorzugte Einbringstellung zu bringen, umfasst das Instrumentarium 10 die bereits erwähnte Fixiereinrichtung 14. Die Fixiereinrichtung 14 ist ausgestaltet in Form einer Hülle 61 mit im Wesentlichen U-förmigem Querschnitt. Dabei weist die Hülle 61 ein erstes Segment 62 sowie ein dazu beabstandetes zweites Segment 63, die jeweils an einem ihrer Enden durch ein drittes Segment 64 miteinander verbunden sind.

Am dritten Segment 64 und durch dieses teilweise begrenzt umfasst die Hülle 61 eine erste Aufnahme 65, in die das Verbindungsglied 48 durch eine Einführöffnung 66 eingeführt werden kann. Die erste Aufnahme 65 ist so bemessen, dass das Verbindungsglied 48 formschlüssig in der Aufnahme platziert werden kann. Dies gibt die Möglichkeit, das Verbindungsglied 48 und damit die erste Implantatkomponente 28 sowie damit wiederum das Implantat 12 mit der Hülle 61 zu verbinden oder, anders ausgedrückt, die Hülle 61 am Implantat 12 anzubringen. In der Aufnahme 65 steht das Verbindungsglied 48 mit dem dritten Segment 64 in Eingriff.

Seitlich neben der ersten Aufnahme 65 umfasst die Hülle 61 eine zweite Aufnahme 67, die obenseitig durch das erste Segment 62 und untenseitig durch das zweite Segment 63 begrenzt ist. Durch eine Einführöffnung 68 kann die zweite Implantatkomponente 29 ungefähr mit der Hälfte ihrer Längserstreckung, bezogen auf den Abstand der Klammer 45 von den Stützelementen 58 bis 60, in die zweite Aufnahme 67 eingeführt werden. Die der zweiten Implantatkomponente 29 zugewandten Wandungen der zweiten Aufnahme 67, die vom ersten und vom zweiten Segment 62 beziehungsweise 63 gebildet werden, sind der Kontur der Stützsegmente 41 beziehungsweise 38 nachempfunden. Dadurch kann die zweite Implantatkomponente 29 längs der Spreizrichtung 51 näherungsweise formschlüssig in der zweiten Aufnahme 67 platziert werden. Dadurch ist die Hülle 61 auch an der zweiten Implantatkomponente 29 gehalten.

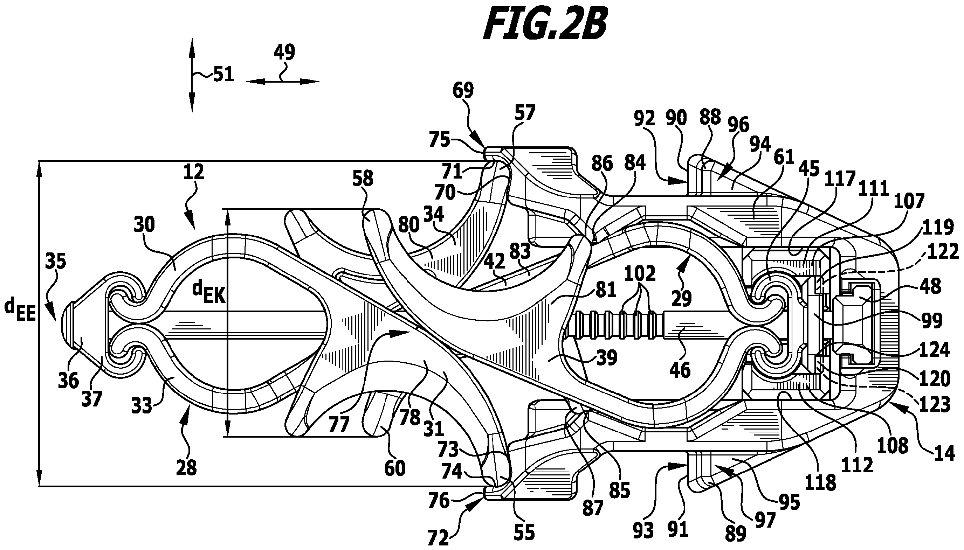

Das erste Segment 62 umfasst an einem dem dritten Segment 64 abgewandten freien Ende 69 ein erstes oberes Anschlagelement 70, welches für den Stützarm 34 entgegen der Spreizrichtung 51 wirksam ist, sowie ein zweites oberes Anschlagelement 71, das für den Stützarm 34 in der Spreizrichtung 51 wirksam ist. In entsprechender Weise umfasst das zweite Segment 63 an einem, dem dritten Segment 64 abgewandten freien Ende 72 für die Stützarme 31 und 32 ein entgegen der Spreizrichtung 51 wirksames erstes unteres Anschlagelement 73 sowie ein für die Stützarme 31 und 32 in der Spreizrichtung 51 wirksames zweites unteres Anschlagelement 74 (Figur 2B).

Die Längen der Segmente 62 und 63 sind so bemessen, dass dann, wenn die Hülle 61 am Implantat 12 angebracht ist, die Stützarme 31 und 32 relativ zum Stützarm 34 gegenüber der Grundstellung längs der Spreizrichtung 51 auseinander gespreizt werden. Die Stützarme 31 und 32 einerseits und der Stützarm 34 andererseits werden insbesondere relativ zueinander vorgespannt. Die Stützarme 31 und 32 und der Stützarm 34 werden auf diese Weise relativ zueinander in einer Weise gespreizt, wie dies auch beim Überführen des Implantates 12 von der Grundstellung in die Spreizstellung erfolgt.

Am ersten oberen Anschlagelement 70 stützt sich der Stützarm 34 mittels des einbringseitigen oberen Stützelementes 57 ab, und am ersten unteren Anschlagelement 73 stützen sich die Stützarme 31 und 32 mittels der einbringseitigen unteren Stützelemente 55 beziehungsweise 56 ab, um die vorstehend beschriebene Spreizung der Stützarme 32 und 33 relativ zum Stützarm 34 sicherzustellen.

Dies definiert eine Einbringstellung des Implantates 12, in der das einbringseitige obere Stützelement 57 von den einbringseitigen unteren Stützelementen 55 und 56 einen Abstand d_{EE} aufweist, der größer ist als der Abstand d_{G} derselben Stützelemente 57 einerseits und 55, 56 andererseits voneinander in der Grundstellung des Implantates 12.

Dies hat zur Folge, dass beim Einbringen des Implantates 12 in den Zwischenwirbelraum die einbringseitigen Stützelemente 55, 56 und 57 Anschläge für den unteren Dornfortsatz 26 beziehungsweise den oberen Dornfortsatz 24 bilden. Dies erleichtert einem Operateur das Einführen des Implantates in den Zwischenwirbelraum, weil es die korrekte Positionierung des Implantates 12 relativ zu den Dornfortsätzen 24 und 26 ermöglicht. Insbesondere kann dadurch auch verhindert werden, dass das Implantat 12 zu weit in den Zwischenwirbelraum eingebracht wird. Dies gibt die Möglichkeit, die Gefahr einer Verletzung des Patienten zu verringern.

Die einbringseitigen Stützelemente 55 und 56 einerseits sowie 57 andererseits können in der Einbringstellung des Implantates 12 zusätzlich am zweiten unteren Anschlagelement 74 beziehungsweise am zweiten oberen Anschlagelement 71 anliegen. Dies sorgt für eine noch zuverlässigere Befestigung der Hülle 61 am Implantat 12 und verhindert außerdem ein übermäßiges Aufspreizen der Stützarme 31 und 32 relativ zum Stützarm 34 und dadurch die Gefahr einer Beschädigung des Implantates 12.

Die Hülle 61 umfasst ferner zwei Knochenanschlagelemente 75 und 76, die am freien Ende 69 des ersten Segmentes 62 beziehungsweise am freien Ende 72 des zweiten Segmentes 63 gebildet sind. Die Knochenanschlagelemente 75 und 76 bilden zusätzlich zu den einbringseitigen Stützelementen 55 bis 57 Anschläge für die Dornfortsätze 24 beziehungsweise 26.

Wie bereits erwähnt, bilden die Stützarme 31, 32 und 34 der ersten Implantatkomponente Aufgleitelemente für die Stützarme 39, 40 beziehungsweise 42 der zweiten Implantatkomponente 29 und umgekehrt. Weil in der Einbringstellung des Implantates 12 die Stützarme 31 und 32 relativ zum Stützarm 34 entlang der Spreizrichtung 51 gespreizt werden, bewegen sich in der Einbringstellung die Stützarme 39 und 40 einerseits und der Stützarm 42 andererseits längs der Spreizrichtung 51, aufgrund der elastischen Ausgestaltung der Stützsegmente 38 und 41, aufeinander zu. Auch in der Einbringstellung des Implantates 12 liegen daher die Stützarme 31 und 39, die Stützarme 32 und 40 sowie die Stützarme 34 und 42 aneinander an.

Dies allerdings hat zur Folge, dass sich der Abstand des körperseitigen unteren Stützelementes 60 von den körperseitigen oberen Stützelementen 58 und 59 in der Einbringstellung des Implantates 12, wo er d_{EK} beträgt, relativ zum Abstand d_{G} verringert. Der Abstand d_{EK} definiert damit die Einbringhöhe des Implantates 12 in der Einbringstellung, welche geringer ist als die Einbringhöhe d_{G} des Implantates 12 in der Grundstellung. Beim Einbringen des Implantates 12 in den Zwischenwirbelraum ist dadurch die Gefahr verringerbar, dass ein Operateur unerwünschterweise an den Dornfortsätzen 24 und 26 oder auch an anderen Körperstrukturen, wie beispielsweise Sehnen, Bändern oder Muskeln, anstößt. Eine Gefahr der Verletzung des Patienten ist dadurch beim Einbringen des Implantates 12 in der Einbringstellung verringerbar.

Durch das Spreizen der Stützarme 31 und 32 relativ zum Stützarm 34 in der Einbringstellung des Implantates 12 mittels der Fixiereinrichtung 14 nähern sich die Stützarme 39 und 40 dem Stützarm 42 längs der Spreizrichtung, wie vorstehend erläutert, gewissermaßen automatisch aneinander an. Dadurch bildet das Implantat 12 eine Kopplungseinrichtung 77 aus, in der die Stützarme 31 und 39, 32 und 40 sowie 34 und 42 jeweils miteinander koppeln. Die Stützarme 31, 32 und 34 mit den einbringseitigen Stützelementen 55, 56 beziehungsweise 57 werden auch als erste Kopplungselemente 78, 79 beziehungsweise 80 der Kopplungseinrichtung 77 bezeichnet. In entsprechender Weise werden die Stützarme 39, 40 und 42 als zweite Kopplungselemente 81, 82 beziehungsweise 83 der Kopplungseinrichtung 77 bezeichnet.

Um zu verhindern, dass sich die zweite Implantatkomponente 29 beim Einbringen in den Körper unerwünschterweise spreizt und sich die Stützarme 39 und 40 relativ zum Stützarm 42 längs der Spreizrichtung 51 bewegen, umfasst die Hülle 41 ein Blockierelement 84 für den Stützarm 39, ein Blockierelement 85 für den Stützarm 42 sowie ein in der Zeichnung nicht dargestelltes Blockierelement für den Stützarm 40 (Figur 2B). Das Blockierelement 84 ist am ersten Segment 62 an einem Rand der zweiten Aufnahme 67 angeordnet, und es kann mit einem einbringseitigen oberen Stützelement 86 des Stützarms 39 zusammenwirken. In entsprechender Weise ist das Blockierelement 85 am zweiten Segment 63 an einem Rand der zweiten Aufnahme 67 angeordnet, und es wirkt mit einem einbringseitigen unteren Stützelement des Stützarmes 42 zusammen.

Die Hülle 61 ist einstückig ausgebildet, und sie besteht aus einem elastisch verformbaren Kunststoffmaterial. Sie kann am Implantat 12 bereits in dessen Auslieferungszustand vormontiert sein Es ist allerdings auch möglich, dass die Hülle 61 erst vor dem Gebrauch des Implantates 12 an diesem angebracht wird.

Weil die Hülle 61 die zweite Implantatkomponente 29 von drei Seiten umgibt, bildet sie ein Schutzelement für das Implantat 12.

Auf den der zweiten Aufnahme 67 abgewandten Seiten der Segmente 62 und 63 umfasst die Hülle 61 zwei keilförmige Vorsprünge 88 bzw. 89. Die Vorsprünge 88 und 89 bilden jeweils ein längs der Spannrichtung 49 wirksames Spreizanschlagelement 90 bzw. 91 aus. Wird auf die Spreizanschlagelemente 90 und 91 eine längs der Spannrichtung 49 wirksame Spreizkraft ausgeübt, greift diese an dem in der ersten Aufnahme 64 formschlüssig gehaltenen Verbindungsglied 48 an und damit an der ersten Implantatkomponente 28. Dadurch bildet die Hülle 61 mit den Vorsprüngen 88 und 89 jeweils ein erstes Spreizlagerelement 92 bzw. 93 zum Spreizen des Implantates 12.

Darüber hinaus umfassen die Vorsprünge 88 und 89 jeweils ein senkrecht zur Spannrichtung 49 sowie senkrecht zur Spreizrichtung 51 wirksames Abziehanschlagelement 94 bzw. 95. Wirkt in dieser Richtung, die eine Abziehrichtung 951 für die Hülle 61 vom Implantat 12 definiert (in Figur 2B senkrecht zur Zeichenebene), kann die Hülle 61 vom Implantat 12 abgezogen werden. Die Vorsprünge 88 und 89 bilden auf diese Weise jeweils ein Abziehlagerelement 96 bzw. 97 zum Abziehen der Hülle 61 vom Implantat 12.
Wie insbesondere aus den Figuren 3, 11 und 12 hervorgeht, umfasst das Implantat 12 eine Verriegelungseinrichtung 98 mit einem plattenförmigen Verriegelungselement 99, welches auf der der Endseite 43 zugewandten Seite der Klammer 45 angeordnet ist und eine senkrecht zur Spannrichtung 49 orientierte Ebene definiert. Mittels des Verriegelungselementes 99 kann das Implantat 12 längs der Spreizrichtung verriegelt werden, indem es von einer Freigabestellung, in welcher die zweite Implantatkomponente 29 relativ zur ersten Implantatkomponente 28 entlang der Spannrichtung 49 beweglich ist, in eine Verriegelungsstellung überführt wird. In der Verriegelungsstellung des Verriegelungselementes 99 ist die zweite Implantatkomponente 29 gegen eine der Spannrichtung 49 gerichtete Bewegung an der ersten Implantatkomponente 28 verriegelt.

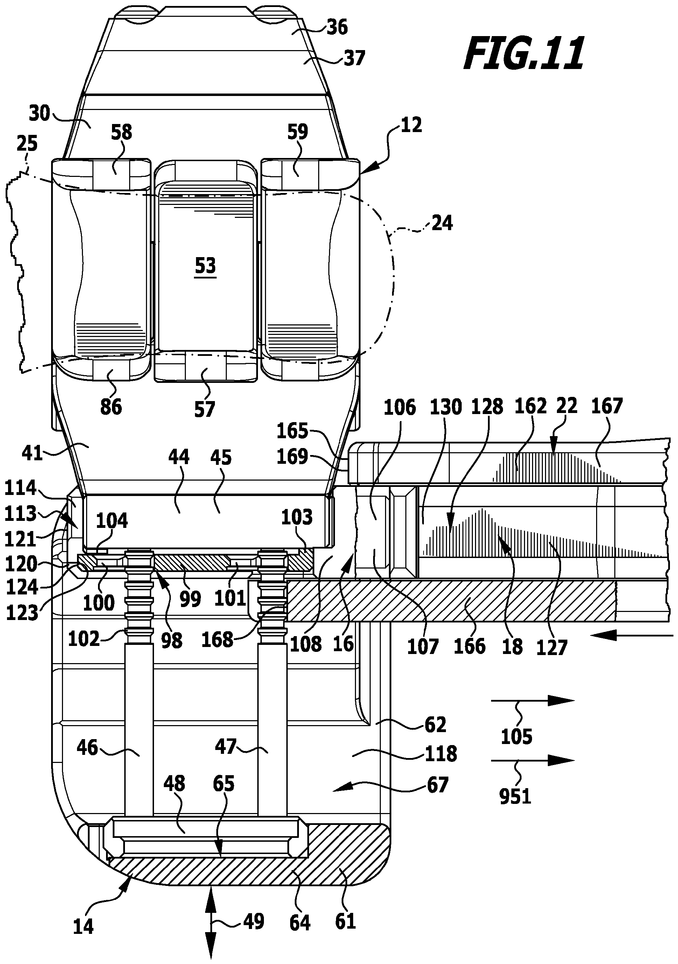

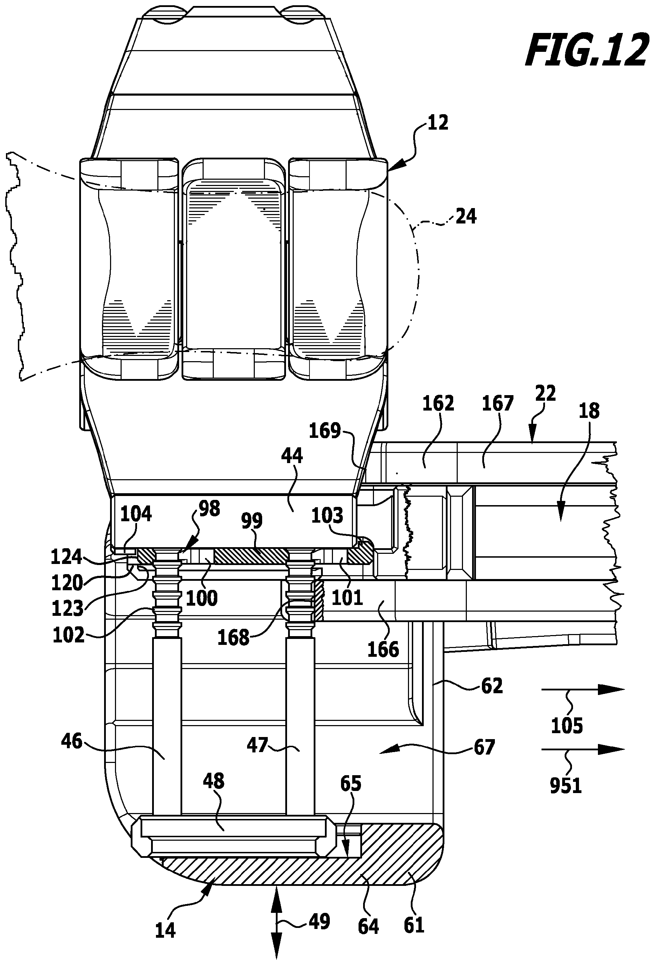

Der Mechanismus, wie dies geschieht, ist in der Druckschrift DE 20 2008 009 344 U1 beschrieben. Während das Verriegelungselement 99 in der Freigabestellung (Figur 11) relativ zu den Spannelementen 46 und 47 längs der Spannrichtung 49 frei beweglich ist, die es an Durchbrechungen 100 bzw. 101 durchgreifen, ist es in der Verriegelungsstellung zwischen an den Spannelementen 46 und 47 jeweils gebildete Verriegelungsglieder in Form von Umfangsrippen 102 eingerückt (Figur 12). Dadurch kann sich das Verriegelungselement 99 in der Verriegelungsstellung an der ersten Implantatkomponente 28 abstützen. Die zweite Implantatkomponente 29 kann sich über die ein Fixierelement bildende Klammer 45 am Verriegelungselement 99 abstützen und ist dadurch gegen eine Bewegung relativ zur ersten Implantatkomponente 28 gesichert.

In der Verriegelungsstellung des Verriegelungselementes 99 können sich das Verriegelungselement 99 und die Klammer 45 zusätzlich mittels Sicherungsgliedern in Form von Vorsprüngen 103 am Verriegelungselement 99 und Vorsprüngen 104 an der Klammer 45 gegenseitig hintergreifen. Dadurch ist das Verriegelungselement 99 in der Verriegelungsstellung gegen eine einer Verriegelungsrichtung 105 entgegen gerichtete Bewegung an der zweiten Implantatkomponente 29 gesichert. Die Verriegelungsrichtung 105 ist senkrecht zur Spannrichtung 49 und senkrecht zur Spreizrichtung 51 sowie parallel zur Abziehrichtung 951 ausgerichtet, und in der Verriegelungsrichtung 105 kann das Verriegelungselement 99 von der Freigabestellung in die Verriegelungsstellung überführt werden.

Die Sicherung des Verriegelungselementes 99 an der zweiten Implantatkomponente 29 erfolgt in der Verriegelungsstellung gewissermaßen automatisch. Dies beruht darauf, dass in der Spreizstellung die Implantatkomponenten 28 und 29 relativ zueinander längs der Spannrichtung 49 vorgespannt sind. Nimmt das Verriegelungselement 99 die Verriegelungsstellung ein, bewegt es sich dadurch relativ zur Klammer 45 längs der Spannrichtung 49, was zu einem automatischen Hintergreifen der Klammer 45 und des Verriegelungselementes 99 mittels der Vorsprünge 103 und 104 führt.

Die eingangs erwähnte Halteeinrichtung 16 für das Implantat 12, dies wird insbesondere aus den Figuren 1 und 3 deutlich, ist klammerförmig ausgestaltet. Die Halteeinrichtung 16, nachfolgend als Adapter 106 bezeichnet, umfasst hierfür ein erstes Halteelement 107 und ein zweites Halteelement 108. Die Halteelemente 107 und 108 sind an einer Endseite 109 des Adapters 106 miteinander über einen stegförmigen Verbindungsabschnitt 110 verbunden, und sie bilden gewissermaßen Arme 111 bzw. 112 des klammerförmigen Adapters 106.

An der der Endseite 109 gegenüberliegenden Seite bildet der Adapter 106 zwischen den Armen 111 und 112 eine Aufnahme 113, in der die Klammer 45 und das Verriegelungselement 99 angeordnet werden können. Die der Klammer 45 und dem Verriegelungselement 99 zugewandten Seiten der Arme 111 und 112 sind deren Kontur jeweils nachempfunden, so dass dann, wenn der Adapter 106 am Implantat 12 montiert ist, die Klammer 45 und das Verriegelungselement 99 längs der Spreizrichtung 51 formschlüssig in der Aufnahme 113 angeordnet sind. Dadurch kann das Implantat 12 sicher am Adapter 106 gehalten werden.

Der Adapter 106 kann am Implantat 12 in dessen Auslieferungszustand vormontiert sein. Es ist allerdings auch möglich, dass der Adapter 106 erst nachträglich am Implantat 12 angebracht wird. Hierzu kann die Aufnahme 113 erweitert werden, nämlich indem die Arme 111 und 112 entlang der Spreizrichtung 51 relativ zueinander gespreizt werden. Dies gibt die Möglichkeit, die Klammer 45 und das Verriegelungselement 99 über eine endseitige Öffnung 114 der Aufnahme 113 in diese einzuführen. Die endseitige Öffnung 114 ist an der der Endseite 109 gegenüberliegenden Endseite 115 des Adapters 106 angeordnet.

Entlang der Spannrichtung 49 ist die endseitige Öffnung 114 so bemessen, dass dann, solange sich das Verriegelungselement 99 in der Freigabestellung befindet und auch in der Verriegelungsstellung, sich in dieser aber noch nicht gegenseitig mit der Klammer 45 hintergreift, die Klammer 45 und das Verriegelungselement 99 nicht durch die endseitige Öffnung 114 aus der Aufnahme 113 entfernt werden können. In diesem Moment sind das Verriegelungselement 99 und die Klammer 45 entlang der Spannrichtung 49 (noch) formschlüssig in der Aufnahme 113 gehalten.

Hintergreifen sich hingegen das Verriegelungselement 99 und die Klammer 45 mittels der Vorsprünge 103 und 104 gegenseitig, können sie durch die endseitige Öffnung 114 hindurch aus der Aufnahme 113 entfernt werden.

Über die Funktion des Haltens des Implantates 12 hinaus bildet der Adapter 106 ein zweites Spreizlagerelement 116 zum Überführen des Implantates 12 von der Einbringstellung in die Spreizstellung. Dies liegt daran, dass er an der Klammer 45, damit an der zweiten Implantatkomponente 29 und damit am Implantat 12 angreift. Wird der Adapter 106 mit einer längs der Spannrichtung 49 wirksamen Kraft beaufschlagt, kann die zweite Implantatkomponente 29 relativ zur ersten Implantatkomponente 28 bewegt und dadurch das Implantat gespreizt werden.

Ist der Adapter 106 zusammen mit der Hülle 61 am Implantat 12 angebracht, ist er in der von der Hülle 61 gebildeten zweiten Aufnahme 67 längs der Spreizrichtung 51 formschlüssig angeordnet. Dies führt dazu, dass die Hülle 61 mit dem ersten Segment 62 ein Führungselement 117 für den ersten Arm 111 und mit dem zweiten Segment 63 ein Führungselement 118 für den Arm 112 ausbildet (Figur 2B). Dies sichert eine besonders zuverlässige Bewegung des Adapters 106 relativ zur Hülle 61 und damit ein zuverlässiges Überführen des Implantates 12 von der Einbringstellung in die Spreizstellung.

Darüber hinaus weist der Adapter 106 eine weitere Funktion auf. Er dient nämlich zum Überführen des Verriegelungselementes 99 von der Freigabestellung in die Verriegelungsstellung. Hierfür umfasst der Adapter 106 zwei Kopplungsglieder 119 und 120, die an den Armen 111 bzw. 112 und speziell an einem Rand 121 der endseitigen Öffnung 114 gebildet sind. Die Kopplungsglieder 119 und 120 sind jeweils ausgestaltet in Form von Mitnehmeranschlägen 122 bzw. 123. Sie können mit einem weiteren vom Verriegelungselement 99 umfassten Kopplungsglied 124, welches stirnseitig am Verriegelungselement 99 ausgebildet ist, zusammenwirken.

Wird auf den Adapter 106 eine Kraft in der Verriegelungsrichtung 105 ausgeübt, koppeln die Kopplungsglieder 119 und 120 mit dem Kopplungsglied 124. Dies bedeutet, dass die Mitnehmeranschläge 122 und 123 das Verriegelungselement 99 mit einer in der Verriegelungsrichtung 105 gerichteten Kraft beaufschlagen, so dass dieses von der Freigabestellung in die Verriegelungsstellung überführt werden kann (Figuren 11 und 12). Dabei wird aufgrund der Größe der endseitigen Öffnung 114 entlang der Spannrichtung 49 sichergestellt, dass weder das Verriegelungselement 99 noch die Klammer 45 sich aus der Aufnahme 113 entfernen. Nimmt das Verriegelungselement 99 seine Verriegelungsstellung ein und hintergreifen sich das Verriegelungselement 99 und die Klammer 45 mittels der Vorsprünge 103 und 104 gegenseitig, kann der Adapter 106 indessen durch weitere Kraftbeaufschlagung in der Verriegelungsrichtung 105 vom Implantat 12 abgezogen werden. Dabei können das Verriegelungselement 99 und die Klammer 45 durch die endseitige Öffnung 114 aus der Aufnahme 113 entfernt werden.

Gemeinsam mit dem Abziehen des Adapters 106 vom Implantat 12 wird dieses somit wie vorstehend beschrieben automatisch in der Spreizstellung verriegelt. Zusätzlich wird das Verriegelungselement 99 automatisch an der Klammer 45 gesichert, so dass es nicht wieder zurück in die Freigabestellung überführt werden kann. Dies sichert eine zuverlässige Handhabung des Implantates 12.

Etwa mittig zwischen den Endseiten 109 und 115 umfasst der Adapter 106 eine Verbindungseinrichtung 125 in Form einer Rasteinrichtung 126. Dies gibt die Möglichkeit, den Adapter 106 mit der eingangs erwähnten ersten Handhabungseinrichtung 18 des Instrumentariums 10 lösbar zu verbinden. Die als Griffarm 127 ausgestaltete erste Handhabungseinrichtung 18 (Figur 4) umfasst ihrerseits ebenfalls eine Verbindungseinrichtung 128 in Form einer Rasteinrichtung 129, die mit der Rasteinrichtung 126 lösbar verbindbar ist. Sie ist an einem Ende 130 des Griffarmes 127 angeordnet. An dem dem Ende 130 gegenüberliegenden Ende umfasst der Griffarm 127 ein Griffelement 131, das von einem Operateur auf einfache Weise ergriffen werden kann.
Etwa mittig zwischen der Verbindungseinrichtung 128 und dem Griffelement 131 umfasst der Griffarm 127 zwei Lagerelemente in Form von Lagerzapfen 132 und 133. An den Lagerzapfen 132 und 133 ist die zweite Handhabungseinrichtung 20, die als Schwenkhebel 134 ausgestaltet ist, am Griffarm 127 um eine von den Lagerzapfen 132 und 133 definierte, parallel zur Spreizrichtung 51 und damit senkrecht zur Spannrichtung 49 ausgerichtete Schwenkachse 135 schwenkbar gelagert.

Der Schwenkhebel 134 ist an seiner dem Implantat 12 zugewandten Seite in zwei Segmente 136 und 137 unterteilt, die zwischen sich den Griffarm 127 aufnehmen können. An den Enden 138 und 139 sind die Segmente 136 bzw. 137 jeweils hakenförmig ausgestaltet. Dort umfassen die Segmente 136 bzw. 137 jeweils ein längs der Spannrichtung 49 wirksames zweites Spreizanschlagelement 140 bzw. 141. Diese können mit den ersten Spreizanschlagelementen 90 bzw. 91 an der Hülle 61 zum Spreizen des Implantates 12 zusammenwirken.

Ebenso umfassen die Segmente 136 und 137 an ihren Enden 138 und 139 jeweils ein zweites Abziehanschlagelement 142 bzw. 143. Diese können mit den ersten Abziehanschlagelementen 95 bzw. 96 der Hülle 61 zu deren Abziehen vom Implantat 12 zusammenwirken.

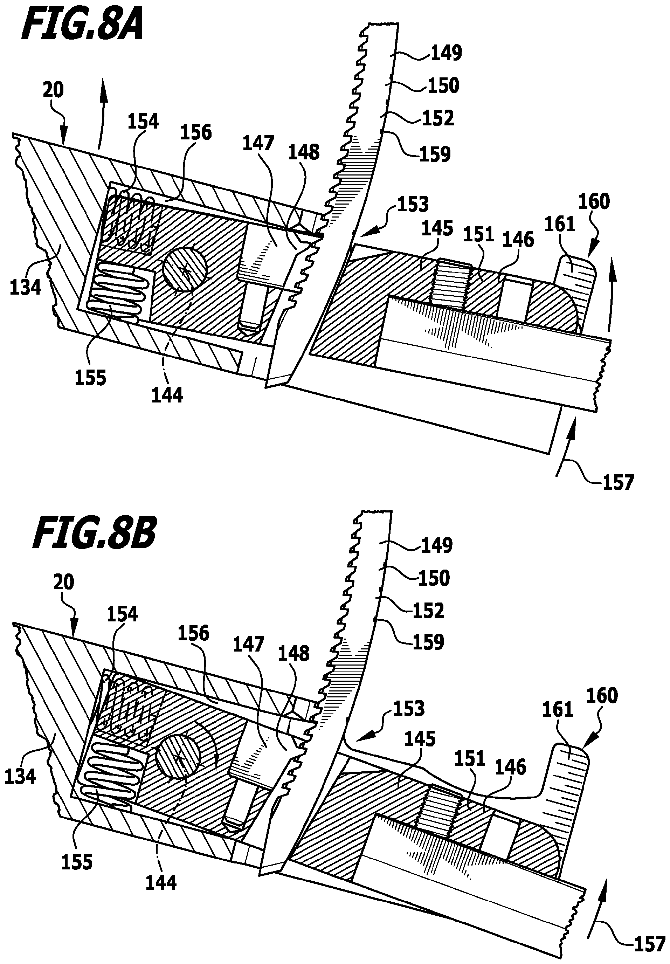

An der den Enden 138 und 139 gegenüberliegenden Seite umfasst der Schwenkhebel 134 einen um eine parallel zur Schwenkachse 135 ausgerichtete Schwenkachse 144 schwenkbaren Betätigungshebel 145. Der Betätigungshebel 145 ist als Rasthebel 146 ausgebildet, und er umfasst ein Rastelement 147 in Form eines Rastzahns 148 (Figuren 8A und 8B).

Der Rastzahn 148 kann mit einem weiteren Rastelement 149 in Form einer am Griffarm 127 gehaltenen Raststange 150 zusammenwirken, um ein Verschwenken des Schwenkhebels 134 relativ zum Griffarm 127 um die Schwenkachse 135 zu sperren oder zu ermöglichen. Aus diesem Grund bildet der Rasthebel 146 ein Sperrelement 151, und die Raststange 150 bildet ein Sperrelement 152. Gemeinsam bilden sie eine Sperreinrichtung 153 des Instrumentariums 10.

Stehen die Sperrelemente 151 und 152 miteinander in Eingriff (Figur 8B), kann der Schwenkhebel 134 nicht relativ zum Griffarm 127 verschwenkt werden. Dies definiert eine Sperrstellung der Sperreinrichtung 153. Die Sperrstellung ist eine Grundstellung der Sperreinrichtung 153, denn der Rasthebel 146 wird mittels zweier elastischer Elemente in Form von Federn 154 und 155, die in einer endseitigen Aufnahme 156 am Schwenkhebel 134 angeordnet sind, so zum Verschwenken um die Schwenkachse 144 beaufschlagt, so dass er automatisch in Eingriff mit der Raststange 150 gelangt.

Erst wenn der Operateur den Rasthebel 146 entgegen der Rückstellkraft der Federn 154 und 155 beaufschlagt, geraten der Rasthebel 146 und die Raststange 150 außer Eingriff (Figur 8A), und die Sperreinrichtung 153 nimmt ihre Lösestellung ein. Dann kann der Schwenkhebel 134 relativ zum Griffarm 127 verschwenkt werden.

Um die Sperreinrichtung 153 von der Sperrstellung in die Lösestellung zu überführen, ist es erforderlich, dass der Operateur den Rasthebel 146 entlang einer Betätigungsrichtung 157 mit einer Betätigungskraft beaufschlagt, entlang welcher Betätigungsrichtung 157 der Schwenkhebel 134 auch zum Spreizen des Implantates 12 relativ zum Griffarm 127 kraftzubeaufschlagen ist. Dies bedeutet, dass das Implantat 12 erst dann von der Einbringstellung in die Spreizstellung überführt werden kann, wenn der Operateur die Sperreinrichtung 153 in die Lösestellung überführt, so dass das Implantat 12 nicht unbeabsichtigterweise gespreizt wird.

Bereits beim Einbringen des Implantats 12 in den Körper verhindert die Sperreinrichtung 153 ein unbeabsichtigtes Spreizen des Implantates 12 infolge eines von Körpergewebe auf die Klammer 37 ausgeübten und der Einführungseinrichtung entgegengerichteten Gegendruckes.

Ferner dient die Vorspannung der Sperrelemente 151 und 152 durch die Federn 154 und 155 relativ zueinander dazu, dass dann, wenn das Implantat 12 etwas gespreizt ist und die Dornfortsätze 24 und 26 eine Kraft auf das Implantat 12 ausüben, das Implantat 12 nicht wieder von der Spreizstellung zurück in die Einbringstellung überführt wird. Stattdessen verbleibt es in der Spreizstellung. Dies erleichtert einem Operateur die Handhabung des Instrumentariums, weil das Implantat 12 vom Operateur schrittweise durch Kraftbeaufschlagung des Rasthebels 146 gespreizt werden kann und der Operateur gewissermaßen nach jedem "Spreiz-Schritt" kontrollieren kann, ob das Implantat 12 ausreichend gespreizt ist, um eine sichere Abstützung der Dornfortsätze 24 und 26 relativ zueinander zu gewährleisten.

Mittels einer an der Raststange 150 angeordneten Anzeigeeinrichtung 158, welche eine Skala 159 umfasst, kann der Operateur kontrollieren, welche Stellung der Griffarm 127 und der Schwenkhebel 134 relativ zueinander einnehmen. Daraus kann der Operateur ableiten, wie weit das Implantat 12 bereits aufgespreizt ist. Es kann insbesondere vorgesehen sein, dass die Skala 159 derart kalibriert ist, dass der Abstand der Stützflächen 53 und 54 voneinander direkt an der Skala 159 abgelesen werden kann.

Eine weitere Anzeigeeinrichtung 160 ist endseitig am Schwenkhebel 134 angeordnet und umfasst eine Skala 161. Anhand der Skala 161 kann der Operateur ermitteln, mit welcher Betätigungskraft er den Rasthebel 146 entgegen der Rückstellkraft der Federn 154 und 155 kraftbeaufschlagt, um das Implantat 12 zu spreizen. Diese Betätigungskraft ist ein Maß für die von den Dornfortsätzen 24 und 26 auf das Implantat 12 eingeleitete und über den Griffarm 127 und den Schwenkhebel 134 abgeleitete Stützkraft. So kann der Operateur anhand der Skala 161 auch die Stützkraft ermitteln. Die Skala 161 kann ebenso wie die Skala 159 kalibriert sein.

Die dritte Handhabungseinrichtung 22 des Instrumentariums 10 ist als Schieber 162 ausgestaltet, der am Griffarm 127 parallel zur Verriegelungsrichtung 105 und zur Abziehrichtung 951 verschieblich gelagert ist. Er umfasst ein erstes Ende 163 mit einem daran angeordneten Betätigungsglied 164 für den Operateur. Dem ersten Ende 163 gegenüber umfasst der Schieber 162 ein zweites Ende 165. Etwa mittig zwischen den Enden 163 und 165 und bis zum Ende 165 bildet der Schieber 162 ein erstes Segment 166. Ferner bildet er ein zweites Segment 167. Diese sind voneinander beabstandet und können den Griffarm 127 zwischen sich aufnehmen sowie ebenso das aus der Hülle 61 herausragende Ende des Adapters 106.

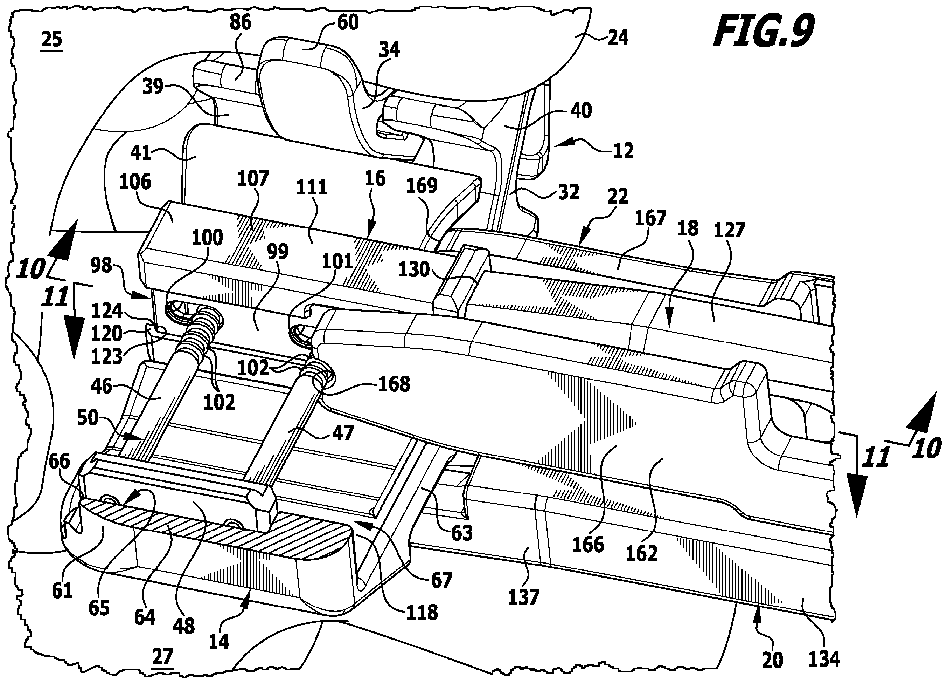

Am zweiten Ende 165 umfasst das erste Segment stirnseitig ein Gegenanschlagelement 168, und in entsprechender Weise umfasst das zweite Segment stirnseitig ein Gegenanschlagelement 169. Die Gegenanschlagelemente 168 und 169 werden als solche bezeichnet, weil über sie eine zum Abziehen der Hülle 61 und des Adapters 106 vom Implantat 12 erforderliche Gegenkraft auf das Implantat 12 eingeleitet werden kann. Dies kann in einer Anlegestellung des Schiebers 162 erfolgen. In der Anlegestellung liegt das Gegenanschlagelement 168 am Zuganker 50 an, insbesondere am Spannelement 47. Das Gegenanschlagelement 169 kann in der Anlegestellung an der zweiten Implantatkomponente 29 anliegen, insbesondere an den Stützsegmenten 38 und 41 (Figur 9).

In einer zurückgezogenen Stellung des Schiebers 152, in die er durch Verschieben relativ zum Griffarm 127 überführt werden kann, weisen die Gegenanschlagelemente 168 und 169 einen Abstand vom Implantat 12 auf.

Die vorstehenden Erläuterungen zusammenfassend, funktioniert das erfindungsgemäße Instrumentarium 10 folgendermaßen:
Die Fixiereinrichtung 14 wird am Implantat 12 angebracht, um dieses von der Grundstellung in die Einbringstellung zu bringen. Die Halteeinrichtung 16 wird am Implantat 12 angebracht. Diese beiden voranstehenden Schritte können bereits werksseitig erfolgen. Typischerweise handelt es sich bei der Halteeinrichtung 16 und bei der Hülle 61 um Wegwerfteile, die nach dem Einsetzen des Implantates 12 in den Körper von diesem gelöst und entsorgt werden.

Der Griffarm 127 wird mittels der Rasteinrichtungen 126 und 129 mit dem Adapter 106 verbunden. Damit kann der Operateur das Implantat 12 am Griffelement 131 halten. Der Schwenkhebel 134 kann so relativ zum Griffarm 127 verschwenkt werden, dass die Segmente 136 und 137 an den keilförmigen Vorsprüngen 88 und 89 aufgleiten. Die Spreizanschlagelemente 90 und 140 sowie 91 und 141 können aneinander zur Anlage geraten. Ebenso können die Abziehanschlagelemente 94 und 142 sowie 95 und 143 aneinander zur Anlage geraten. Der Schieber 162 nimmt eine zurückgezogene Stellung ein.

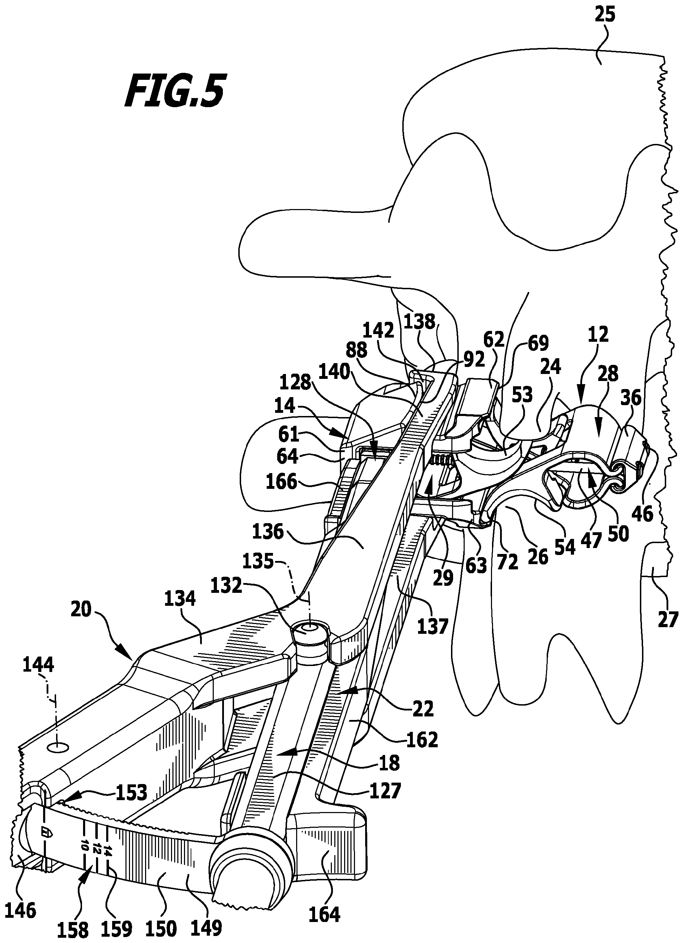

Das Implantat 12 ist mittels der Blockierelemente 84 und 85 sowie der Sperreinrichtung 153 gegen unbeabsichtigtes Aufspreizen gesichert. Es kann (Figur 5) vom Operateur in den Zwischenwirbelraum eingebracht werden. Die Knochenanschlagelemente 75 und 76 sowie die einbringseitigen Stützelemente 55, 56 und 57 bilden Anschlagelemente für die Dornfortsätze 24 und 26 und begrenzen dadurch die Einbringtiefe des Implantates 12 in den Zwischenwirbelraum.

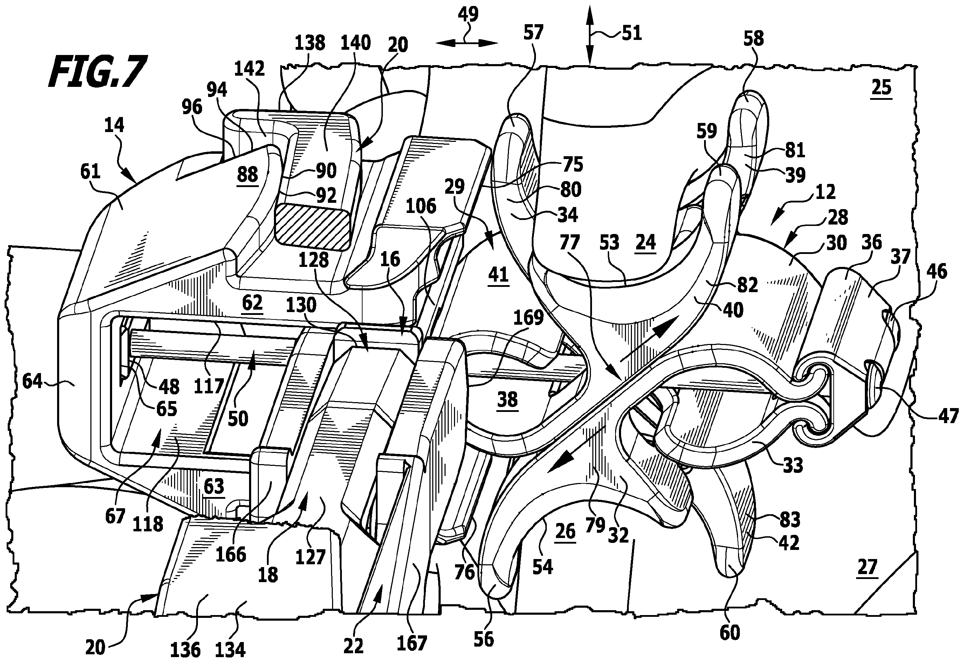

Durch Kraftbeaufschlagung des Rasthebels 146 entlang der Betätigungsrichtung 157 kann der Schwenkhebel 134 relativ zum Griffarm 127 verschwenkt werden. Die Spreizanschlagelemente 90 und 140 sowie 91 und 141 wirken zusammen, um auf die erste Implantatkomponente 28 eine Spreizkraft einzuleiten. Die zum Spreizen erforderliche Gegenkraft wird mittels des Griffarmes 127 über den Adapter 106 auf die zweite Implantatkomponente 29 eingeleitet. Das Implantat 12 wird von der Einbringstellung in die Spreizstellung überführt (Figuren 6 und 7). Die Dornfortsätze 24 und 26 werden mittels der Stützflächen 53 bzw. 54 relativ zueinander abgestützt.

Der Operateur kann den Griffarm 127 und damit den mit diesem gekoppelten Schwenkhebel 134 mit nur einer Hand halten und bedienen. Mit der anderen Hand kann er den Schieber 162 am Betätigungsglied 164 von der zurückgezogenen Stellung in die Anlegestellung überführen. Die Abziehanschlagelemente 94 und 142 sowie 95 und 143 wirken zum Abziehen der Hülle 61 vom Implantat 12 zusammen. Die Gegenkraft wird über die Gegenanschlagelemente 168 und 169 auf das Implantat 12 eingeleitet.

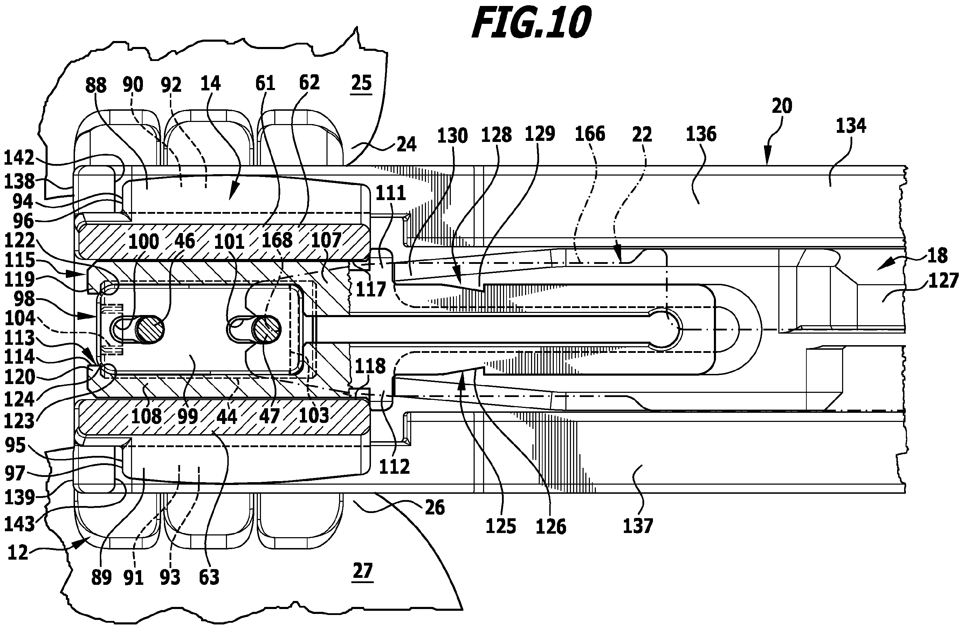

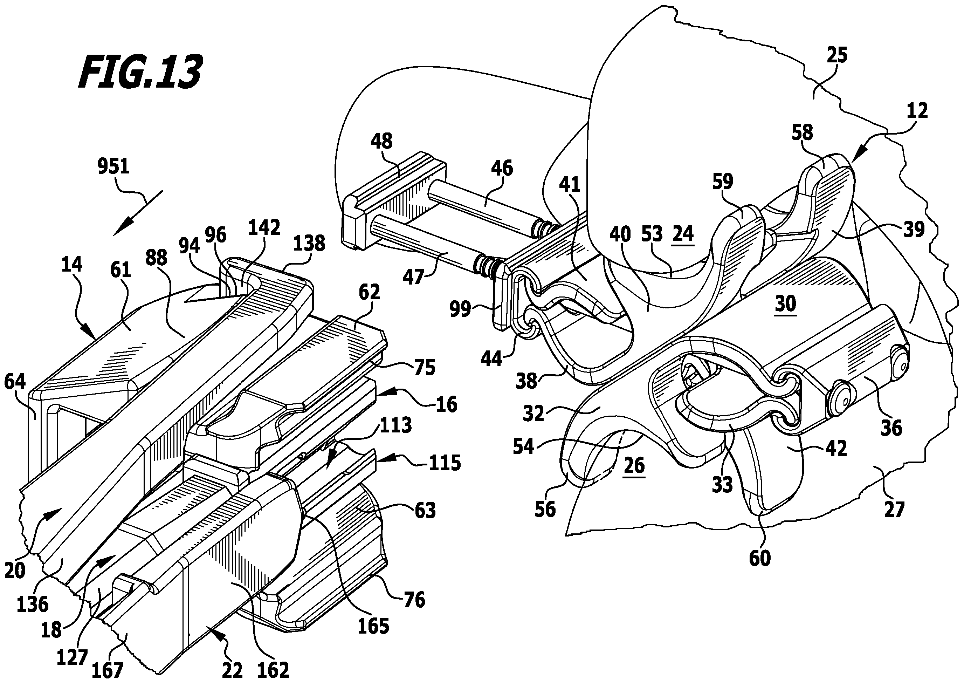

Zugleich kann der Adapter 106 von der Klammer 37 und vom Verriegelungselement 99 abgezogen werden. Die hierfür erforderliche Gegenkraft kann ebenso über die Gegenanschlagelemente 168 und 169 auf das Implantat 12 eingeleitet werden, und die Zugkraft greift am Griffarm 127 an. Beim Abziehen des Adapters 106 vom Implantat 12 kann das Verriegelungselement 99 wie vorstehend beschrieben von der Freigabestellung in die Verriegelungsstellung überführt und damit das Implantat 12 automatisch verriegelt werden (Figuren 9 bis 12). Durch die endseitige Öffnung 114 hindurch können die Klammer 37 und das Verriegelungselement 99 aus der Aufnahme 113 entfernt werden. Somit können die Hülle 61 und der Adapter 106 gemeinsam, am Griffarm 127 und am Schwenkhebel 134 gehalten, vom Implantat 12 abgezogen werden (Figur 13). Das verriegelte Implantat 12 verbleibt in der Spreizstellung bestimmungsgemäß zwischen den Dornfortsätzen 24 und 26.

Der Adapter 106 und die Hülle 61 können vom Griffarm 127 bzw. vom Schwenkhebel 134 gelöst und entsorgt oder wieder verwendet werden.

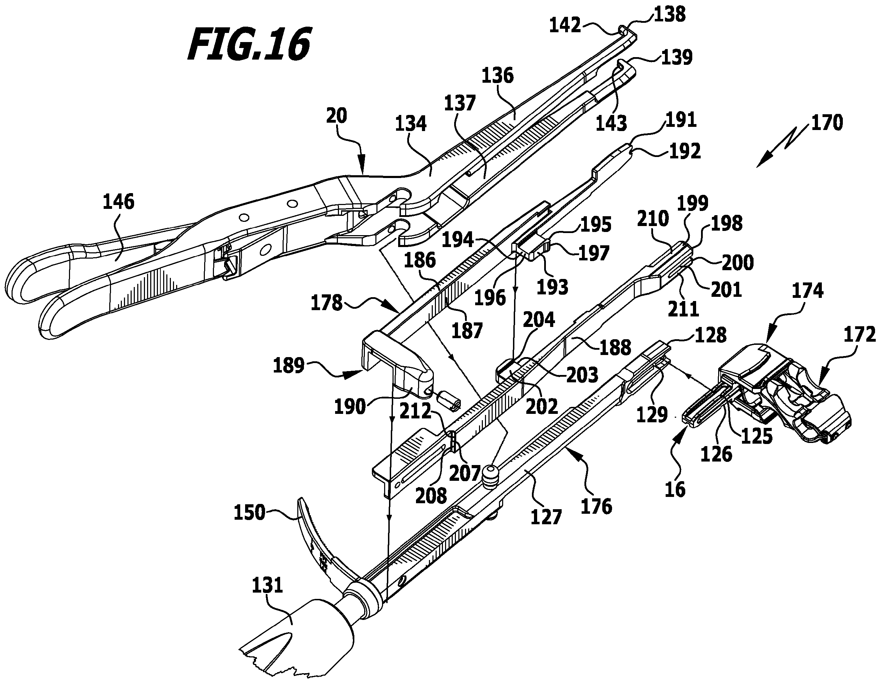

Eine weitere Ausführungsform eines nicht erfindungsgemäßen chirurgischen Instrumentariums ist in Figur 16 teilweise in Explosionsdarstellung gezeigt und dort mit dem Bezugszeichen 170 belegt. Es umfasst ein Implantat 172, eine Fixiereinrichtung 174, die Halteeinrichtung 16, eine zur ersten Handhabungseinrichtung 18 funktionsgleiche erste Handhabungseinrichtung 176, die zweite Handhabungseinrichtung 20 sowie eine dritte Handhabungseinrichtung 178.

Das Instrumentarium 170 ist in seinen einzelnen Bestandteilen weitestgehend identisch aufgebaut zum Instrumentarium 10, und für gleiche und gleichwirkende Merkmale der Instrumentarien 10 und 170 werden daher dieselben Bezugszeichen benutzt. Die mit dem Instrumentarium 10 erzielbaren Vorteile können mit dem Instrumentarium 170 ebenfalls erzielt werden. Nachfolgend wird lediglich auf die wesentlichen Unterschiede der einzelnen Komponenten der Instrumentarien 10 und 170 eingegangen.

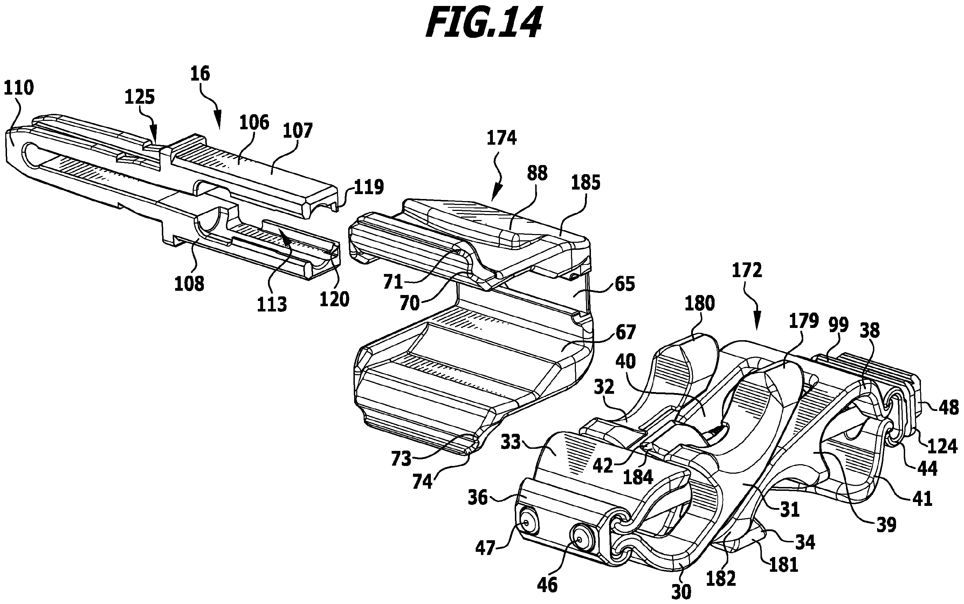

Anders als beim Implantat 12 sind beim Implantat 172 die Orientierungen der Stützarme 31, 32, 34, 39, 40 und 42 längs der Spreizrichtung 51 umgekehrt ausgebildet. Dies bedeutet, dass die Stützarme 31 und 32 einbringseitige obere Stützelemente 179 beziehungsweise 180 umfassen und der Stützarm 34 ein einbringseitiges unteres Stützelement 181. Die Stützarme 39 und 40 umfassen körperseitige untere Stützelemente 182 beziehungsweise 183, und der Stützarm 42 umfasst ein köperseitiges oberes Stützelement 184 (Figuren 14 bis 15B). Die Stützarme 31, 32, 34, 39, 40 und 42 bilden somit Träger der Stützelemente 179, 180, 181, 182, 183 beziehungsweise 184.

In der Einbringstellung des Implantates 172 liegen dementsprechend an den oberen Anschlagelementen 70 und 71 der Fixiereinrichtung 174 die einbringseitigen oberen Stützelemente 179 und 180 an, und an den unteren Anschlagelementen 73 und 74 der Fixiereinrichtung 174 liegt das einbringseitige untere Stützelement 181 an.

Anders als die Hülle 61 weist die von der Fixiereinrichtung 174 gebildete Hülle 185 nicht die Knochenanschlagelemente 75 und 76 auf.

Ebenso im Unterschied zur Hülle 61 umfasst die Hülle 185 nicht die Blockierelemente 84 und 85.

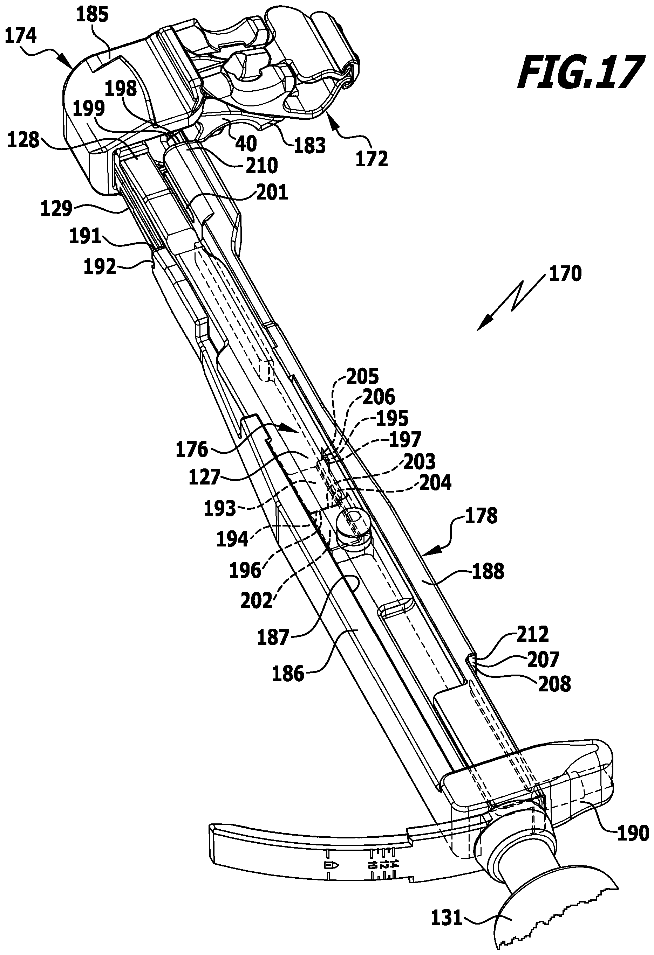

Die dritte Handhabungseinrichtung 178 ist ähnlich zur dritten Handhabungseinrichtung 22 aufgebaut und als Schieber 186 ausgestaltet mit einem ersten Segment 187 und einem zweiten Segment 188. Die Segmente 187 und 188 sind grundsätzlich relativ zueinander entlang des Griffarmes 127 verschieblich ausgestaltet und können auf verschiedene Weise miteinander koppeln:
Wie insbesondere aus den Figuren 16 und 17 deutlich wird, umfasst das erste Segment 187 am ersten Ende 189 des Schiebers 186 ein Betätigungsglied 190. An dem dem ersten Ende 189 gegenüberliegenden zweiten Ende 191 des ersten Segmentes 187 umfasst dieses ein Gegenanschlagelement 192, das in einer Anlegestellung des Schiebers 186 an den Zuganker 50 anlegbar ist.

Etwa mittig zwischen den Enden 189 und 191 weist das erste Segment 187 einen Vorsprung 193 in Richtung des zweiten Segmentes 188 auf. Der Vorsprung 193 bildet zwei Koppelelemente 194 und 195 in Form eines Mitnehmeranschlages 196 beziehungsweise einer Rastnase 197. Diese sind jeweils längs der Verrieglungsrichtung 105 wirksam. Das zweite Segment 188 umfasst an einem dem ersten Ende 189 gegenüberliegenden zweiten Ende 198 ein Paar von Gegenanschlagelementen 199 und 200, die voneinander durch einen schlitzförmigen Zwischenraum 201 getrennt sind. Etwa mittig zwischen den Enden 189 und 198 umfasst das zweite Segment 188 einen Vorsprung 202, der ein Koppelelement 203 in Form eines Gegenanschlages 204 für den Mitnehmeranschlag 196 ausbildet. Darüber hinaus umfasst das zweite Segment 188 nahe dem Vorsprung 202 ein Koppelelement 205 in Form eines Vorsprunges 206 für die Rastnase 197. Ferner bildet das zweite Segment 188 ein Koppelelement 207 in Form einer Schulter 208, die mit dem Betätigungsglied 290 zusammenwirken kann.

Zum Einbringen des Implantates 172 in den Körper werden beim Instrumentarium 170 zunächst der Griffarm 127 am Adapter 106 befestigt und der Schwenkhebel 134 an der Hülle 185 adaptiert (Figur 17, in der der Schwenkhebel 134 nicht dargestellt ist). Der Schieber 186 nimmt eine zurückgezogene Stellung ein, in der die Gegenanschlagelemente 192, 199 und 200 einen Abstand vom Implantat 172 aufweisen und die Rastnase 197 am Vorsprung 206 anliegt.

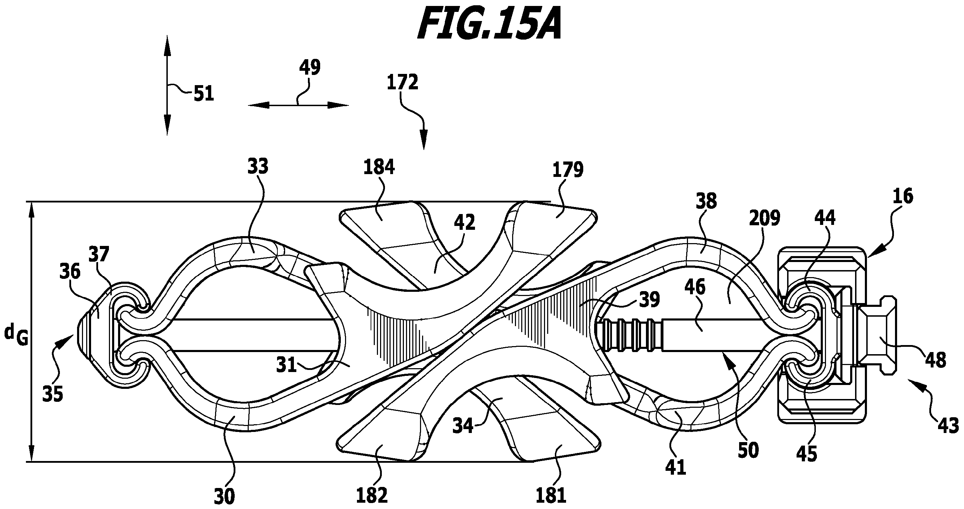

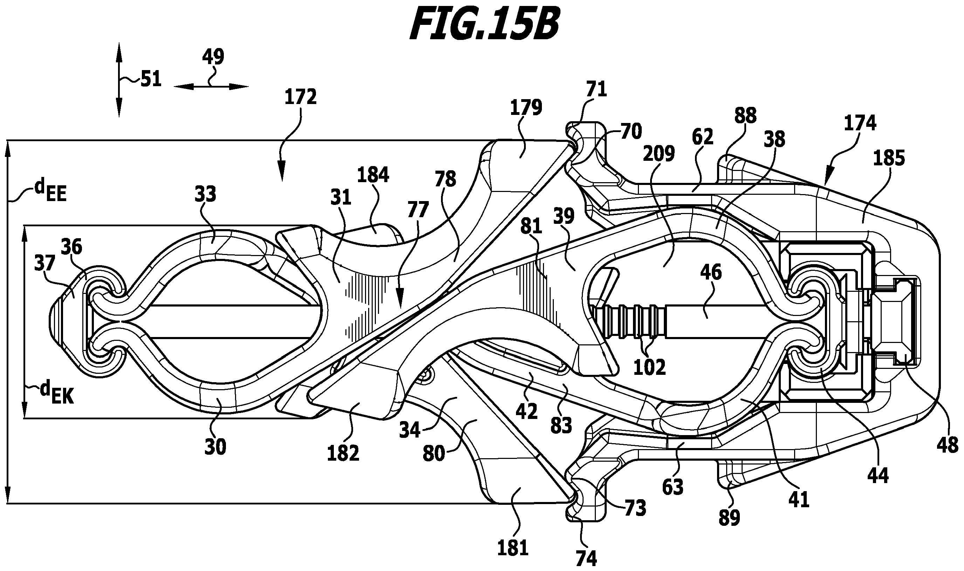

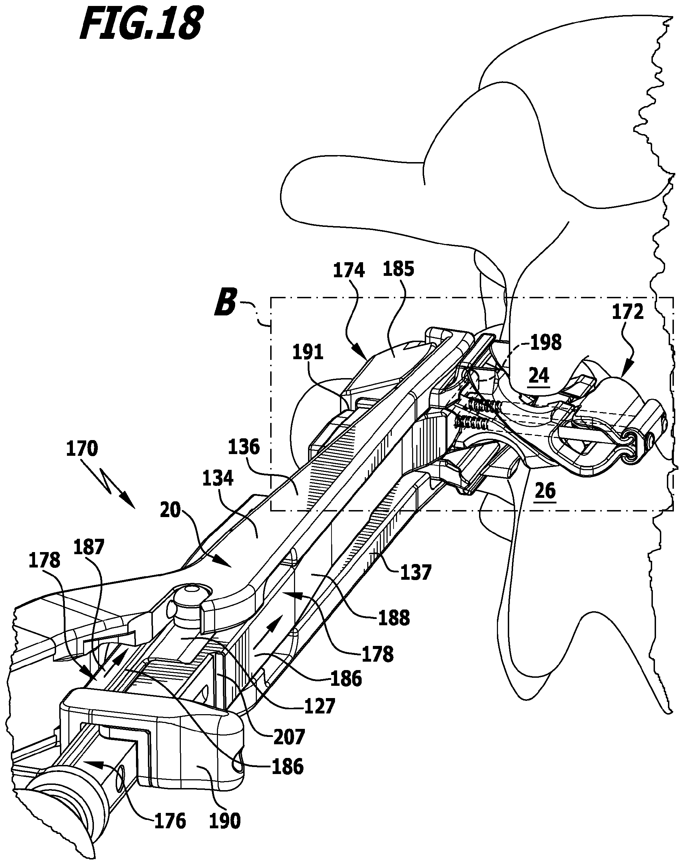

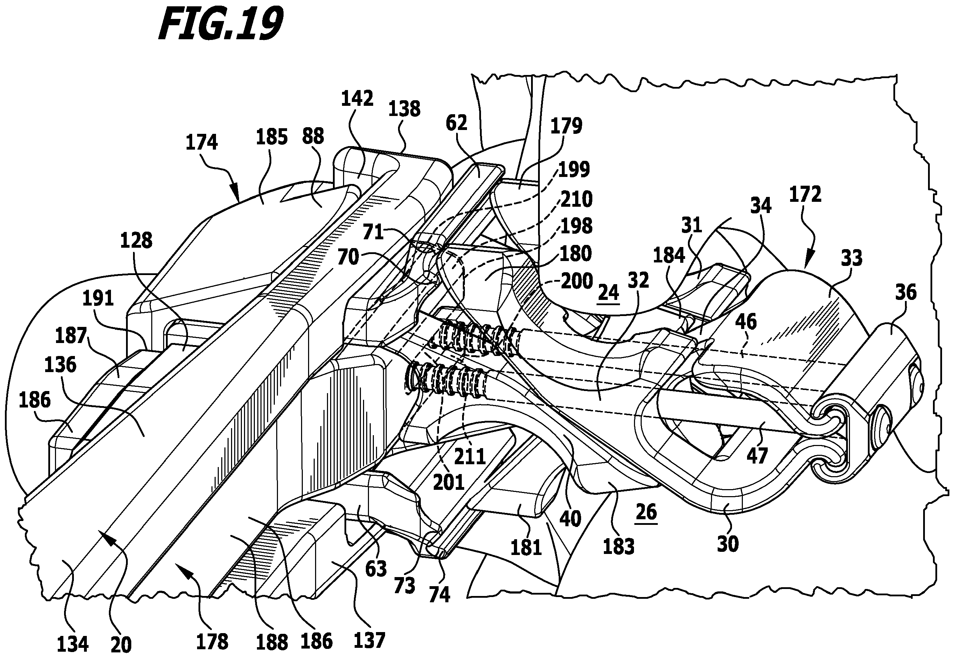

Vor dem Einbringen des Implantates 172 in den Körper wird der Schieber 186 vom Operateur am Betätigungsglied 190 mit einer auf das Implantat 172 gerichteten Kraft beaufschlagt. Die Segmente 187 und 188 koppeln mittels der Rastnase 197 und des Vorsprunges 206. Dies erlaubt es, die Gegenanschlagelemente 199 und 200 in einen zwischen den Stützsegmenten 38 und 41 des Implantates 172 gebildeten Zwischenraum 209 (Figuren 15A und 15B) einzuführen. Dabei greifen die Spannelemente 46 und 47 in den Zwischenraum 201 zwischen den Gegenanschlagelementen 199 und 200 ein (Figuren 18 und 19). Im Zwischenraum 209 ist das zweite Segment 188 mit den Gegenanschlagelementen 199 und 200 formschlüssig anordenbar, so dass die Stützarme 39 und 40 nicht relativ zum Stützarm 42 längs der Spreizrichtung 51 gespreizt werden können. Aus diesem Grund bilden die Gegenanschlagelemente 199 und 200 Blockierelemente 210 beziehungsweise 211, mit denen ein unerwünschtes Aufspreizen des Implantates 172 verhindert werden kann. Dies ist auch dadurch bedingt, dass durch den formschlüssigen Eingriff des zweiten Segments 188 in den Zwischenraum 209 der Adapter 106 gegen eine Bewegung relativ zur Hülle 185 gesichert ist. Die Stellung des Schiebers 186, bei der das zweite Segment 188 in den Zwischenraum 209 eingreift, wird als Blockierstellung des Schiebers 186 bezeichnet.

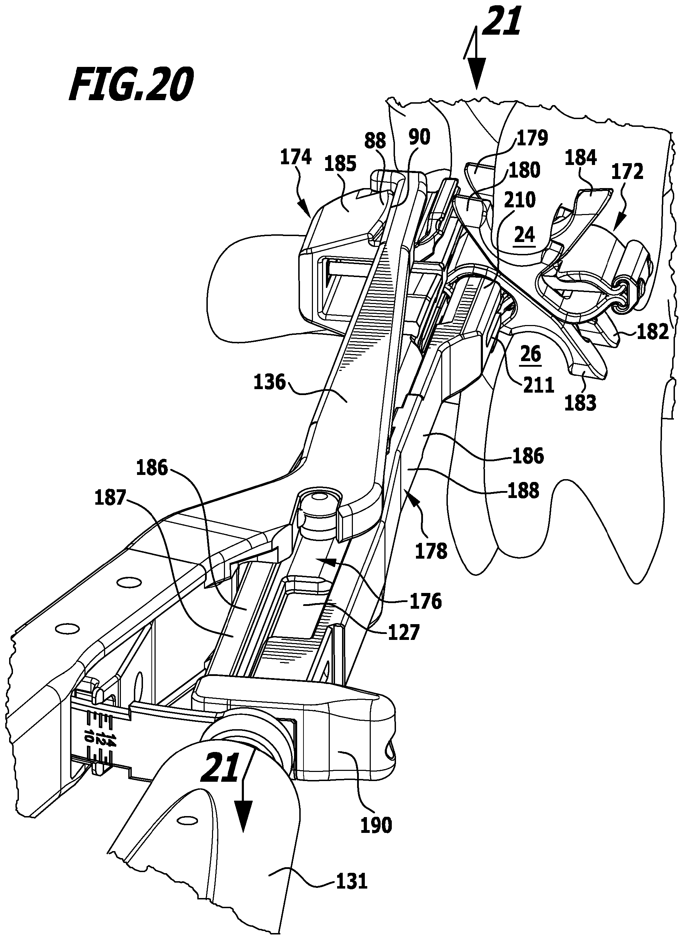

Damit das Implantat 172 nach dem Einbringen in den Zwischenwirbelraum gespreizt werden kann, muss der Operateur das Betätigungsglied 190 mit einer in der Verriegelungsrichtung 105 gerichteten Kraft beaufschlagen. Dabei koppeln die Segmente 187 und 188 mittels des Mitnehmeranschlages 196 und des Gegenanschlages 204. Dadurch kann der Schieber 186 wieder in die zurückgezogene Stellung überführt werden. Das Implantat 172 kann daraufhin in entsprechender zu der vorstehenden erläuterten Weise wie das Implantat 12 von der Einbringstellung in die Spreizstellung überführt werden (Figur 20).

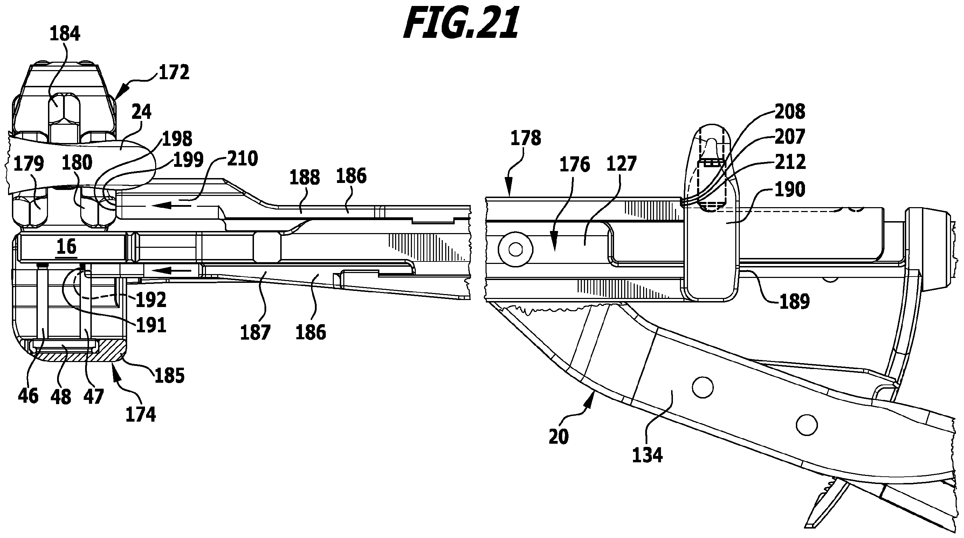

Um nach dem Spreizen des Implantates 172 die Hülle 185 und den Adapter 106 von diesem zu lösen, kann der Operateur das Betätigungsglied 190 erneut mit einer auf das Implantat 172 gerichteten Kraft beaufschlagen. Dies führt dazu, dass das zweite Segment 188 mit den Gegenanschlagelementen 199 und 200 zur Anlage an der zweiten Implantatkomponente 29 gelangt und sich an dieser abstützt. Unter der Wirkung der Betätigungskraft des Operateurs und der Gegenkraft des Implantates 172 kann die Rastnase 197 vom Vorsprung 206 entkoppeln. Dadurch kann das erste Segment 187 relativ zum zweiten Segment 188 so lange längs des Griffarmes 127 verschoben werden, bis das Betätigungsglied 190 zur Anlage an die Schulter 208 gelangt (Figur 21). Ist dies der Fall, liegt das erste Segment 187 mit dem Gegenanschlagelement 192 am Spannelement 47 an.

Die Schulter 208 bildet einen Mitnehmeranschlag 212, so dass bei weiterer Beaufschlagung des Betätigungsgliedes 190 mit einer auf das Implantat 172 gerichteten Kraft auf dieses eine Gegenkraft zum Abziehen der Hülle 185 und des Adapters 106 eingeleitet werden kann. Damit können beim Instrumentarium 170 die Hülle 185 und der Adapter 106, wie vorstehend für den Fall des Instrumentariums 10 beschrieben, vom Implantat 172 abgezogen und dieses ferner automatisch verriegelt werden.

## Patentansprüche

1. Chirurgisches Instrumentarium (10; 170), umfassend ein Implantat (12; 172) zur gegenseitigen Abstützung eines oberen Dornfortsatzes (24) eines ersten Wirbelkörpers (25) mittels einer oberen Stützfläche (53) und eines unteren Dornfortsatzes (26) eines zweiten Wirbelkörpers (27) mittels einer unteren Stützfläche (54), wobei das Implantat (12; 172) eine erste Implantatkomponente (28) und eine relativ zu dieser entlang einer Spannrichtung (49) beweglich ausgebildete zweite Implantatkomponente (29) umfasst, um das Implantat (12; 172) ausgehend von einer Grundstellung in einer quer zur Spannrichtung (49) ausgerichteten Spreizrichtung (51) in eine Spreizstellung zu überführen, in der die obere Stützfläche (53) und die untere Stützfläche (54) einen größeren Abstand voneinander aufweisen als in der Grundstellung, und wobei das Implantat (12; 172) mindestens ein einbringseitiges oberes Stützelement (57; 179, 180) und mindestens ein einbringseitiges unteres Stützelement (55, 56; 181) zum einbringseitigen lateralen Abstützen des oberen Dornfortsatzes (24) bzw. des unteren Dornfortsatzes (26) in der Spreizstellung des Implantates (12; 172) umfasst, **dadurch gekennzeichnet, dass** das Instrumentarium (10; 170) eine Fixiereinrichtung (14; 174) für das Implantat (12; 172) umfasst zum Spreizen des mindestens einen einbringseitigen oberen Stützelementes (57; 179, 180) und des mindestens einen einbringseitigen unteren Stützelementes (55, 56; 181) relativ zueinander entlang der Spreizrichtung (51), mittels welcher Fixiereinrichtung (14; 174) das Implantat (12; 172) ausgehend von der Grundstellung in eine Einbringstellung bringbar ist, in der das mindestens eine einbringseitige obere Stützelement (57; 179, 180) und das mindestens eine einbringseitige untere Stützelement (55, 56; 181) einen größeren Abstand (dEE) relativ zueinander aufweisen als in der Grundstellung, und aus der das Implantat (12; 172) durch Bewegen der zweiten Implantatkomponente (29) relativ zur ersten Implantatkomponente (28) entlang der Spannrichtung (49) in die Spreizstellung überführbar ist.

2. Chirurgisches Instrumentarium nach Anspruch 1, **dadurch gekennzeichnet, dass** die Fixiereinrichtung (14; 174) mindestens einen entgegen der Spreizrichtung (51) wirksamen ersten oberen Anschlag (70) für das mindestens eine einbringseitige obere Stützelement (57; 179, 180) oder einen dieses umfassenden Träger (34; 31, 32) umfasst oder ausbildet und/oder dass die Fixiereinrichtung (14; 174) mindestens einen entgegen der Spreizrichtung (51) wirksamen ersten unteren Anschlag (73) für das mindestens eine einbringseitige untere Stützelement (55, 56; 181) oder einen dieses umfassenden Träger (31, 32; 34) umfasst oder ausbildet.

3. Chirurgisches Instrumentarium nach einem der voranstehenden Ansprüche, **dadurch gekennzeichnet, dass** die Fixiereinrichtung (14; 174) mindestens einen in der Spreizrichtung (51) wirksamen zweiten oberen Anschlag (71) für das mindestens eine einbringseitige obere Stützelement (57; 179, 180) oder einen dieses umfassenden Träger (34; 31, 32) umfasst oder ausbildet und/oder dass die Fixiereinrichtung (14; 174) mindestens einen in der Spreizrichtung (51) wirksamen zweiten unteren Anschlag (74) für das mindestens eine einbringseitige untere Stützelement (55, 56; 181) oder einen dieses umfassenden Träger (31, 32; 34) umfasst oder ausbildet.

4. Chirurgisches Instrumentarium nach einem der voranstehenden Ansprüche, **dadurch gekennzeichnet, dass** die erste Implantatkomponente (28) das mindestens eine einbringseitige obere Stützelement (57; 179, 180) und das mindestens eine einbringseitige untere Stützelement (55, 56; 181) umfasst.

5. Chirurgisches Instrumentarium nach einem der voranstehenden Ansprüche, **dadurch gekennzeichnet, dass** das Implantat (12; 172) mindestens ein körperseitiges oberes Stützelement (58, 59; 184) und mindestens ein körperseitiges unteres Stützelement (60; 182, 183) zum körperseitigen lateralen Abstützen des oberen Dornfortsatzes (24) bzw. des unteren Dornfortsatzes (26) in der Spreizstellung des Implantates (12; 172) umfasst.

6. Chirurgisches Instrumentarium nach Anspruch 5, **dadurch gekennzeichnet, dass** die zweite Implantatkomponente (29) das mindestens eine körperseitige obere Stützelement (58, 59; 184) und das mindestens eine körperseitige untere Stützelement (60; 182, 183) umfasst.

7. Chirurgisches Instrumentarium nach Anspruch 5 oder 6, **dadurch gekennzeichnet, dass** das chirurgische Instrumentarium (10; 170) eine Kopplungseinrichtung (77) umfasst mit mindestens einem mit einem einbringseitigen Stützelement (55, 56, 57; 179, 180, 181) gekoppelten ersten Kopplungselement (78, 79, 80) sowie mit mindestens einem mit einem körperseitigen Stützelement (58, 59, 60; 182, 183, 184) gekoppelten zweiten Kopplungselement (81, 82, 83), das in der Einbringstellung des Implantates (12; 172) mit dem mindestens einen ersten Kopplungselement (78, 79, 80) zusammenwirkt zur Verringerung des Abstandes (dEK) des mindestens einen körperseitigen oberen Stützelementes (58, 59; 184) und des mindestens einen körperseitigen unteren Stützelementes (60; 182, 183) voneinander relativ zu deren Abstand (dG) voneinander in der Grundstellung des Implantates (12; 172).

8. Chirurgisches Instrumentarium nach Anspruch 7, **dadurch gekennzeichnet, dass** das mindestens eine erste Kopplungselement (78, 79, 80) als ein das einbringseitige Stützelement (55, 56, 57; 179, 180, 181) an einem freien Ende umfassender erster Träger (31, 32, 34) ausgebildet ist, der ein längs der Spreizrichtung (51) wirksames Aufgleitelement für das mindestens eine zweite Kopplungselement (81, 82, 83) ausbildet, und dass das mindestens eine zweite Kopplungselement (81, 82, 83) als ein das körperseitige Stützelement (58, 59, 60; 182, 183, 184) an einem freien Ende umfassender zweiter Träger (39, 40, 42) ausgebildet ist, der ein längs der Spreizrichtung (51) wirksames Aufgleitelement für das mindestens eine erste Kopplungselement (78, 79, 80) ausbildet.

9. Chirurgisches Instrumentarium nach einem der voranstehenden Ansprüche, **dadurch gekennzeichnet, dass** die Fixiereinrichtung (14; 174) einen im wesentlichen U-förmigen Querschnitt aufweist.

10. Chirurgisches Instrumentarium nach einem der voranstehenden Ansprüche, **dadurch gekennzeichnet, dass** die Fixiereinrichtung (14; 174) einstückig ausgebildet ist.

11. Chirurgisches Instrumentarium nach einem der voranstehenden Ansprüche, **dadurch gekennzeichnet, dass** die Fixiereinrichtung (14; 174) aus einem verformbaren Material gefertigt ist.

12. Chirurgisches Instrumentarium nach einem der voranstehenden Ansprüche, **dadurch gekennzeichnet, dass** die Fixiereinrichtung (14; 174) am Implantat (12; 172) vormontiert ist.

13. Chirurgisches Instrumentarium nach einem der voranstehenden Ansprüche, **dadurch gekennzeichnet, dass** eine am Implantat (12; 172) montierte Fixiereinrichtung (14; 174) von diesem lösbar ist.

14. Chirurgisches Instrumentarium nach einem der voranstehenden Ansprüche, **dadurch gekennzeichnet, dass** die Fixiereinrichtung (14; 174) ein erstes Abziehlagerelement (96, 97) zum Abziehen der Fixiereinrichtung (14; 174) vom Implantat (12; 172) ausbildet.

15. Chirurgisches Instrumentarium nach einem der voranstehenden Ansprüche, **dadurch gekennzeichnet, dass** die Fixiereinrichtung (14; 174) ein erstes Spreizlagerelement (92, 93) zum Überführen des Implantates (12; 172) von der Einbringstellung in die Spreizstellung ausbildet.

## Claims

1. Surgical apparatus (10; 170) comprising an implant (12; 172) for mutually supporting an upper spinous process (24) of a first vertebral body (25) by means of an upper supporting surface (53) and a lower spinous process (26) of a second vertebral body (27) by means of a lower supporting surface (54), wherein the implant (12; 172) comprises a first implant component (28) and a second implant component (29) which is configured such as to be movable relative thereto along a clamping direction (49) in order to transfer the implant (12; 172) out of a basic position in a spreading direction (51) that is oriented transversely relative to the clamping direction (49) into a spread position in which the upper supporting surface (53) and the lower supporting surface (54) are a greater spacing from each other than in the basic position, and wherein the implant (12; 172) comprises at least one introduction-side upper supporting element (57; 179, 180) and at least one introduction-side lower supporting element (55, 56; 181) that are used for laterally supporting the upper spinous process (24) and the lower spinous process (26) on the introduction-side, respectively, in the spread position of the implant (12; 172), **characterized in that** the apparatus (10; 170) comprises a fixing device (14; 174) for the implant (12; 172) that is used for spreading the at least one introduction-side upper supporting element (57; 179, 180) and the at least one introduction-side lower supporting element (55, 56; 181) relative to each other along the spreading direction (51) and by means of which fixing device (14; 174) the implant (12; 172) is movable from the basic position into an introduction position in which the at least one introduction-side upper supporting element (57; 179, 180) and the at least one introduction-side lower supporting element (55, 56; 181) are at a greater spacing (dEE) relative to each other than in the basic position, and from which the implant (12; 172) is transferable into the spread position by moving the second implant component (29) relative to the first implant component (28) along the clamping direction (49).

2. Surgical apparatus in accordance with Claim 1, **characterized in that** the fixing device (14; 174) comprises or forms at least one first upper stop (70) that acts in a direction opposed to the spreading direction (51) for the at least one introduction-side upper supporting element (57; 179, 180) or a carrier (34; 31, 32) incorporating it and/or **in that** the fixing device (14; 174) comprises or forms at least one first lower stop (73) that acts in a direction opposed to the spreading direction (51) for the at least one introduction-side lower supporting element (55, 56; 181) or a carrier (31, 32; 34) incorporating it.

3. Surgical apparatus in accordance with any of the preceding Claims, **characterized in that** the fixing device (14; 174) comprises or forms at least one second upper stop (71) which acts in the spreading direction (51) for the at least one introduction-side upper supporting element (57; 179, 180) or a carrier (31, 32; 34) incorporating it and/or **in that** the fixing device (14; 174) comprises or forms at least one second lower stop (74) which acts in the spreading direction (51) for the at least one introduction-side lower supporting element (55, 56; 181) or a carrier (31, 32; 34) incorporating it.

4. Surgical apparatus in accordance with any of the preceding Claims, **characterized in that** the first implant component (28) comprises the at least one introduction-side upper supporting element (57; 179, 180) and the at least one introduction-side lower supporting element (55, 56; 181).

5. Surgical apparatus in accordance with any of the preceding Claims, **characterized in that** the implant (12; 172) comprises at least one body-side upper supporting element (58, 59; 184) and at least one body-side lower supporting element (60; 182, 183) for laterally supporting of the upper spinous process (24) and the lower spinous process (26) on the body-side, respectively, in the spread position of the implant (12; 172).

6. Surgical apparatus in accordance with Claim 5, **characterized in that** the second implant component (29) comprises the at least one body-side upper supporting element (58, 59; 184) and the at least one body-side lower supporting element (60; 182, 183).

7. Surgical apparatus in accordance with Claim 5 or 6, **characterized in that** the surgical apparatus (10; 170) comprises a coupling device (77) having at least one first coupling element (78, 79, 80) which is coupled to an introduction-side supporting element (55, 56, 57; 179, 180, 181) and also at least one second coupling element (81, 82, 83) which is coupled to a body-side supporting element (58, 59, 60; 182, 183, 184) and which, in the introduction position of the implant (12; 172), cooperates with the at least one first coupling element (78, 79, 80) for decreasing the spacing (dEK) of the at least one body-side upper supporting element (58, 59; 184) and the at least one body-side lower supporting element (60; 182, 183) from each other relative to their spacing (dG) from each other in the basic position of the implant (12; 172).

8. Surgical apparatus in accordance with Claim 7, **characterized in that** the at least one first coupling element (78, 79, 80) is formed as a first carrier (31, 32, 34) which comprises at a free end the introduction-side supporting element (55, 56, 57; 179, 180, 181) and forms a slide-on element which acts along the spreading direction (51) for the at least one second coupling element (81, 82, 83), and **in that** the at least one second coupling element (81, 82, 83) is formed as a second carrier (39, 40, 42) which comprises at a free end the body-side supporting element (58, 59, 60; 182, 183, 184) and forms a slide-on element which acts along the spreading direction (51) for the at least one first coupling element (78, 79, 80).

9. Surgical apparatus in accordance with any of the preceding Claims, **characterized in that** the fixing device (14; 174) has a substantially U-shaped cross section.

10. Surgical apparatus in accordance with any of the preceding Claims, **characterized in that** the fixing device (14; 174) is formed in one piece manner.

11. Surgical apparatus in accordance with any of the preceding Claims, **characterized in that** the fixing device (14; 174) is made from a deformable material.

12. Surgical apparatus in accordance with any of the preceding Claims, **characterized in that** the fixing device (14; 174) is pre-mounted on the implant (12; 172).

13. Surgical apparatus in accordance with any of the preceding Claims, **characterized in that** a fixing device (14; 174) mounted on the implant (12; 172) is releasable therefrom.

14. Surgical apparatus in accordance with any of the preceding Claims, **characterized in that** the fixing device (14; 174) forms a first extraction bearing element (96, 97) for extracting the fixing device (14; 174) from the implant (12; 172).

15. Surgical apparatus in accordance with any of the preceding Claims, **characterized in that** the fixing device (14; 174) forms a first spreading bearing element (92, 93) for transferring the implant (12; 172) from the introduction position into the spread position.

## Revendications

1. Instrumentation chirurgicale (10 ; 170) comprenant un implant (12 ; 172) destiné à assurer l'appui réciproque d'une apophyse supérieure (24) d'un premier corps de vertèbre (25) au moyen d'une surface d'appui supérieure (53), et d'une apophyse inférieure (26) d'un deuxième corps de vertèbre (27) au moyen d'une surface d'appui inférieure (54), instrumentation chirurgicale
dans laquelle l'implant (12 ; 172) comprend un premier composant d'implant (28) et un deuxième composant d'implant (29), qui est réalisé mobile par rapport au premier le long d'une direction de serrage (49), en vue de transférer l'implant (12 ; 172), à partir d'une position de base, selon une direction d'expansion (51) orientée transversalement à la direction de serrage (49), dans une position d'expansion dans laquelle la surface d'appui supérieure (53) et la surface d'appui inférieure (54) présentent une distance d'espacement réciproque plus grande que dans la position de base,
et dans laquelle l'implant (12 ; 172) comprend au moins un élément d'appui supérieur (57 ; 179, 180) du côté insertion et au moins un élément d'appui inférieur (55, 56 ; 181) du côté insertion, pour assurer l'appui latéral du côté insertion de l'apophyse supérieure (24) et respectivement de l'apophyse inférieure (26) dans la position d'expansion de l'implant (12 ; 172),
**caractérisée en ce que** l'instrumentation (10 ; 170) comprend un dispositif de fixation (14 ; 174) pour l'implant (12 ; 172) pour assurer l'expansion dudit au moins un élément d'appui supérieur (57 ; 179, 180) du côté insertion et dudit au moins un élément d'appui inférieur (55, 56 ; 181) du côté insertion, l'un par rapport à l'autre, le long de la direction d'expansion (51), dispositif de fixation (14 ; 174) au moyen duquel l'implant (12 ; 172) peut être amené de la position de base dans une position d'insertion dans laquelle ledit au moins un élément d' appui supérieur (57 ; 179, 180) du côté insertion et ledit au moins un élément d'appui inférieur (55, 56 ; 181) du côté insertion présentent une distance d'espacement (dEE) l'un par rapport à l'autre, qui est plus grande que dans la position de base, et de laquelle l'implant (12 ; 172) peut être transféré dans la position d'expansion par déplacement du deuxième composant d'implant (29) par rapport au premier composant d'implant (28) le long de la direction de serrage (49).

2. Instrumentation chirurgicale selon la revendication 1, **caractérisée en ce que** le dispositif de fixation (14 ; 174) comporte ou forme au moins une première butée supérieure (70) agissant à l'encontre de la direction d'expansion (51) et destinée audit au moins un élément d'appui supérieur (57 ; 179, 180) du côté insertion ou à un support (34 ; 31, 32) comportant celui-ci, et/ou **en ce que** le dispositif de fixation (14 ; 174) comporte ou forme au moins une première butée inférieure (73) agissant à l'encontre de la direction d'expansion (51) et destinée audit au moins un élément d' appui inférieur (55, 56 ; 181) du côté insertion ou à un support (31, 32 ; 34) comportant celui-ci.

3. Instrumentation chirurgicale selon l'une des revendications précédentes, **caractérisée en ce que** le dispositif de fixation (14 ; 174) comporte ou forme au moins une deuxième butée supérieure (71) agissant dans la direction d'expansion (51) et destinée audit au moins un élément d'appui supérieur (57 ; 179, 180) du côté insertion ou à un support (34 ; 31, 32) comportant celui-ci, et/ou **en ce que** le dispositif de fixation (14 ; 174) comporte ou forme au moins une deuxième butée inférieure (74) agissant dans la direction d'expansion (51) et destinée audit au moins un élément d'appui inférieur (55, 56 ; 181) du côté insertion ou à un support (31, 32 ; 34) comportant celui-ci.

4. Instrumentation chirurgicale selon l'une des revendications précédentes, **caractérisée en ce que** le premier composant d'implant (28) comprend ledit au moins un élément d'appui supérieur (57 ; 179, 180) du côté insertion, et ledit au moins un élément d'appui inférieur (55, 56 ; 181) du côté insertion.

5. Instrumentation chirurgicale selon l'une des revendications précédentes, **caractérisée en ce que** l'implant (12 ; 172) comprend au moins un élément d'appui supérieur (58, 59 ; 184) du côté corps, et au moins un élément d'appui inférieur (60 ; 182, 183) du côté corps destinés à assurer un appui latéral côté corps de l'apophyse supérieure (24) et respectivement de l'apophyse inférieure (26), dans la position d'expansion de l'implant (12 ; 172).

6. Instrumentation chirurgicale selon la revendication 5, **caractérisée en ce que** le deuxième composant d'implant (29) comporte ledit au moins un élément d'appui supérieur (58, 59 ; 184) du côté corps, et ledit au moins élément d'appui inférieur (60 ; 182, 183) du côté corps.

7. Instrumentation chirurgicale selon la revendication 5 ou la revendication 6, **caractérisée en ce que** l'instrumentation chirurgicale (10 ; 170) comprend un dispositif de couplage (77) comprenant au moins un premier élément de couplage (78, 79, 80) couplé à un élément d'appui du côté insertion (55, 56, 57 ; 179, 180, 181), ainsi qu'au moins un deuxième élément de couplage (81, 82, 83), qui est couplé à un élément d'appui du côté corps (58, 59, 60 ; 182, 183, 184) et qui dans la position d'insertion de l'implant (12 ; 172) interagit avec ledit au moins un premier élément de couplage (78, 79, 80) pour réduire la distance d'espacement réciproque (dEK) dudit au moins un élément d'appui supérieur (58, 59 ; 184) du côté corps et dudit au moins un élément d'appui inférieur (60 ; 182, 183) du côté corps, par rapport à leur distance d'espacement réciproque (dG) dans la position de base de l'implant (12 ; 172).

8. Instrumentation chirurgicale selon la revendication 7, **caractérisée en ce que** ledit au moins un premier élément de couplage (78, 79, 80) est réalisé en tant que premier support (31, 32, 34) qui englobe, à une extrémité libre, l'élément d'appui du côté insertion (55, 56, 57 ; 179, 180, 181), et qui forme un élément de glissement agissant le long de la direction d'expansion (51) pour ledit au moins un deuxième élément de couplage (81, 82, 83), et **en ce que** ledit au moins un deuxième élément de couplage (81, 82, 83) est réalisé en tant que deuxième support (39, 40, 42) qui englobe, à une extrémité libre, l'élément d'appui du côté corps (58, 59, 60 ; 182, 183, 184), et qui forme un élément de glissement agissant le long de la direction d'expansion (51) pour ledit au moins un premier élément de couplage (78, 79, 80).

9. Instrumentation chirurgicale selon l'une des revendications précédentes, **caractérisée en ce que** le dispositif de fixation (14 ; 174) présente une section transversale sensiblement en forme de U.

10. Instrumentation chirurgicale selon l'une des revendications précédentes, **caractérisée en ce que** le dispositif de fixation (14 ; 174) est réalisé d'un seul tenant.

11. Instrumentation chirurgicale selon l'une des revendications précédentes, **caractérisée en ce que** le dispositif de fixation (14 ; 174) est fabriqué en un matériau déformable.

12. Instrumentation chirurgicale selon l'une des revendications précédentes, **caractérisée en ce que** le dispositif de fixation (14 ; 174) est prémonté sur l'implant (12 ; 172).

13. Instrumentation chirurgicale selon l'une des revendications précédentes, **caractérisée en ce qu'**un dispositif de fixation (14 ; 174) monté sur l'implant (12 ; 172) peut être détaché de celui-ci.

14. Instrumentation chirurgicale selon l'une des revendications précédentes, **caractérisée en ce que** le dispositif de fixation (14 ; 174) forme un premier élément de palier de retrait (96, 97) pour retirer le dispositif de fixation (14 ; 174) de l'implant (12 ; 172).

15. Instrumentation chirurgicale selon l'une des revendications précédentes, **caractérisée en ce que** le dispositif de fixation (14 ; 174) forme un premier élément de palier d'expansion (92, 93) pour transférer l'implant (12 ; 172) de la position d'insertion à la position d'expansion.
